(19) Europäisches Patentamt
European Patent Office
Office européen des brevets

(11) Veröffentlichungsnummer: **0 191 474**
**A1**

# (12) EUROPÄISCHE PATENTANMELDUNG

(21) Anmeldenummer: 86101771.3

(22) Anmeldetag: 12.02.86

(51) Int. Cl.4: C 07 D 403/12, A 61 K 31/515

(30) Priorität: 15.02.85 CH 704/85

(43) Veröffentlichungstag der Anmeldung: 20.08.86 Patentblatt 86/34

(84) Benannte Vertragsstaaten: AT BE CH DE FR IT LI LU NL SE

(71) Anmelder: CIBA-GEIGY AG, Klybeckstrasse 141, CH-4002 Basel (CH)

(72) Erfinder: Sturm, Elmar, Dr., Klusstrasse 66, CH-4147 Aesch (CH)
Erfinder: Gallay, Jean Jacques, Dr., Blumenrain 19, CH-4312 Magden (CH)
Erfinder: Pissiotas, Georg, Dr., Am Sonnenrain 71, D-7850 Lörrach (DE)

(74) Vertreter: Zumstein, Fritz, Dr. et al, Bräuhausstrasse 4, D-8000 München 2 (DE)

## (54) Barbitursäurederivate.

(57) Neue Phenylcarbamoylbarbitursäurederivate der allgemeinen Formel

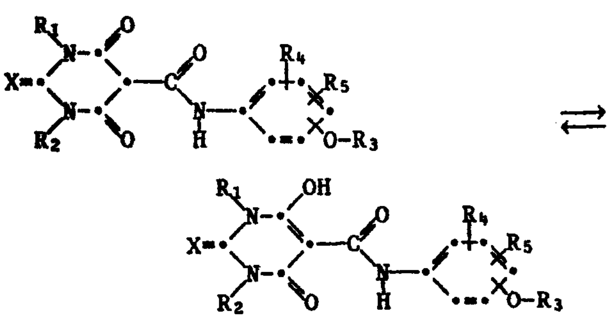

einschliesslich ihrer tautomeren Formen und Salze, worin

$R_1$ $C_1$–$C_4$-Alkyl, $C_1$–$C_3$-Alkoxy, Allyl oder $C_3$–$C_6$-Cycloalkyl,

$R_2$ $C_1$–$C_4$-Alkyl oder Allyl,

$R_3$ einen unsubstituierten oder substituierten heteroaromatischen 6-gliedrigen Ring, welcher 2 oder 3 Stickstoffatome enthält oder einen benzokondensierten, unsubstituierten oder substituierten heteroaromatischen 6-gliedrigen Ring, welcher 1 bis 3 Stickstoffatome enthält,

$R_4$ und $R_5$ unabhängig voneinander Wasserstoff, $C_1$–$C_4$-Alkyl, $C_1$–$C_4$-Alkoxy oder Halogen-$C_1$–$C_3$-Alkyl und

X ein Sauerstoff- oder Schwefelatom bedeuten.

Die Verbindungen können als Wirkstoffe zur Bekämpfung tierparasitärer Helminthen eingesetzt werden.

EP 0 191 474 A1

0 191 474

CIBA-GEIGY AG
Basel (Schweiz)

5-15267/+

Barbitursäurederivate

Die vorliegende Erfindung betrifft neue substituierte 5-Phenylcarbamoylbarbitursäurederivate mit anthelmintischer Wirksamkeit, Mittel, die diese Wirkstoffe als Aktivsubstanzen enthalten, sowie die Verwendung der Wirkstoffe bzw. der Mittel zur Bekämpfung von Helminthen, insbesondere von Nematoden, Cestoden und Trematoden in Haus- und Nutztieren, vor allem in Warmblütern, insbesondere in Säugetieren.

Die Erfindung betrifft ferner die Herstellung der neuen Wirkstoffe und der sie enthaltenden Mittel sowie neue Zwischenprodukte und ihre Herstellung.

Die neuen Verbindungen entsprechen der allgemeinen Formel I

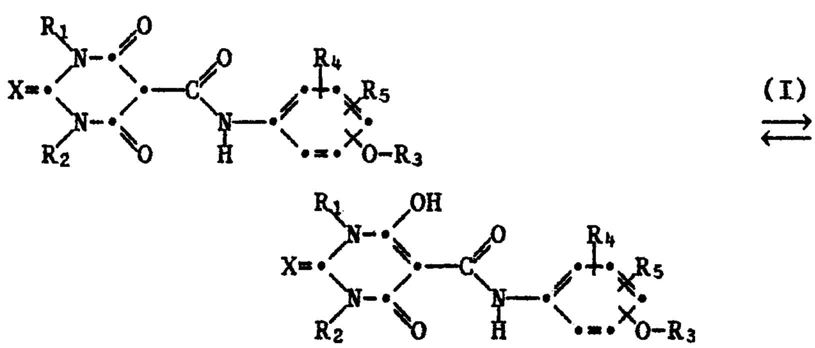

(I)

einschliesslich ihrer tautomeren Formen und Salze, worin
$R_1$ $C_1$-$C_4$-Alkyl, $C_1$-$C_3$-Alkoxy, Allyl oder $C_3$-$C_6$-Cycloalkyl,
$R_2$ $C_1$-$C_4$-Alkyl oder Allyl,

$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und

X ein Sauerstoff- oder Schwefelatom bedeuten.

Die vorliegende Erfindung erstreckt sich auch auf N-oxyde von Vebindungen der Formel I.

Als Substituenten $R_3$ kommen beispielsweise unsubstituierte oder substituierte Vertreter der Gruppe Pyrimidinyl, Triazinyl, Pyrazinyl, Pyridazinyl, Chinolyl, Isochinolyl, Phthalazinyl, Chinoxalyl, Chinazolinyl, Cinnolinyl und Benzotriazinyl in Betracht.

Als Substituenten der vorgenannten Ringe und Ringsysteme sind $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cycloalkyl oder Phenyl zu nennen.

Als Substituent $R_3$ kommen beispielsweise folgende cyclische Reste in Betracht:

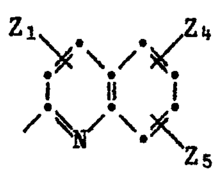 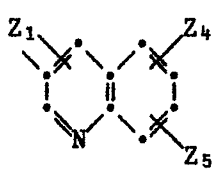 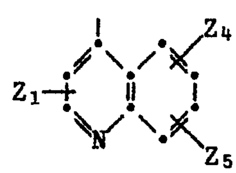 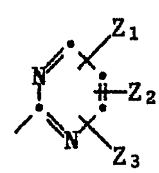

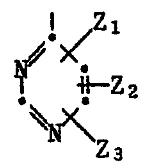 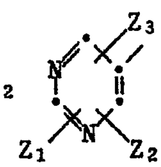 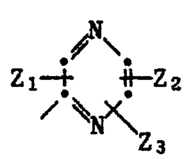 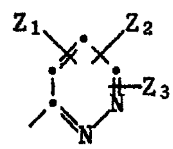 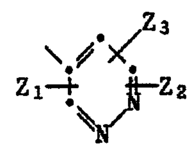

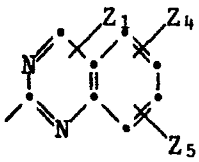 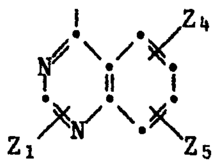 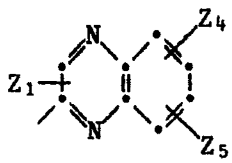 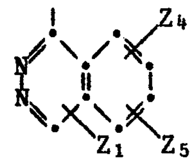

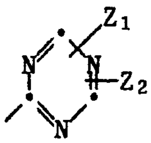 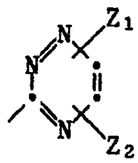 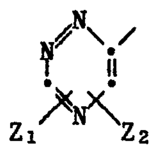 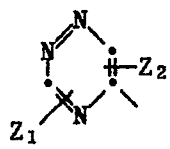 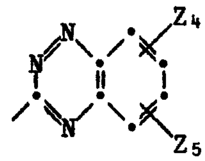

In den vorgenannten Resten stehen $Z_1$, $Z_2$, $Z_3$, $Z_4$ und $Z_5$ unabhängig voneinander für Wasserstoff, $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cycloalkyl oder Phenyl, wobei $Z_1$, $Z_2$ und $Z_3$ am heteroaromatischen, $Z_4$ und $Z_5$ am ankondensierten homoaromatischen Ring stehen. Besonders erwähnenswerte Reste $Z_1$, $Z_2$, $Z_3$, $Z_4$ und $Z_5$ sind Halogen, vorzugsweise Chlor, sowie Trifluormethyl und Methylthio.

Unter Alkyl als Substituent oder Teil eines Substituenten sind Methyl, Aethyl, n-Propyl, Isopropyl, n-Butyl, Isobutyl, sek.-Butyl und tert.-Butyl zu verstehen. Diese Reste werden gemeinsam auch als Niederalkyl bezeichnet.

Als Cycloalkyl kommen Cyclopropyl, Cyclobutyl, Cyclopentyl und Cyclohexyl in Betracht, vorzugsweise Cyclopropyl.

Unter Halogen als Substituent oder Teil eines Substituenten sind Fluor, Chlor, Brom oder Jod zu verstehen, bevorzugt Fluor und Chlor, insbesondere Chlor.

Halogenalkylreste sind Methyl mit 1 bis 3 Halogenatomen, wie Chlormethyl und Fluormethyl, und $C_2$-$C_5$-Alkyl mit 1 bis 5 Halogenatomen. Bevorzugt sind Alkylreste mit 3 Halogenatomen.

Beispiele für $C_1$-$C_4$-Alkoxy sind Methoxy, Aethoxy, n-Propoxy, Isopropoxy, für $C_1$-$C_4$-Alkylthio Methylthio und n-Propylthio, für Halogen-$C_1$-$C_4$-Alkoxy Chlormethoxy, Fluormethoxy, 2-Chloräthoxy, 2,2-Dichloräthoxy, 3-Fluor-n-propoxy, und 2,2,2-Trifluoräthoxy, und für Halogen-$C_1$-$C_4$-Alkylthio Fluormethylthio, Chlormethylthio, 1,2-Dichloräthylthio und 2,2-Difluormethylthio.

Unter einem Di-($C_1$-$C_3$-Alkyl)-aminorest ist im Rahmen der vorliegenden Erfindung eine Aminogruppe zu verstehen, in welcher die beiden Wasserstoffatome durch zwei gleiche oder verschiedene Alkylgruppen mit 1 bis 3 Kohlenstoffatomen ersetzt sind. Bevorzugt ist die Dimethylaminogruppe.

Zu den in Frage kommenden Salzen der Verbindungen der Formel I zählen beispielsweise die Alkalimetall-, Ammonium- oder Aminsalze, wobei Natrium-, Kalium-, Ammonium- oder Alkylaminsalze, besonders Triäthylaminsalze, bevorzugt sind.

Von besonderem Interesse sind Verbindungen der Formel I, in denen $R_1$ $C_1$-$C_4$-Alkyl, $C_1$-$C_3$-Alkoxy oder $C_3$-$C_6$-Cycloalkyl und $R_2$ $C_1$-$C_4$-Alkyl bedeuten, und insbesondere solche, in denen $R_1$ für Methyl oder Methoxy und $R_2$ für Methyl stehen.

Eine interessante Gruppe stellen Verbindungen der Formel I dar, in denen $R_1$ $C_1$-$C_4$-Alkyl, vorzugsweise Methyl oder Aethyl, Methoxy, Allyl oder Cyclopropyl, $R_2$ Methyl, $R_3$ einen unsubstituierten oder ein- bis dreifach durch Substituenten der Gruppe $C_1$-$C_4$-Alkyl, vorzugsweise Methyl, Isopropyl und tert.-Butyl, Methoxy, Methylamino, Dimethylamino, Methylthio, Trifluormethyl, Halogen, vorzugsweise Chlor, oder Phenyl substituierten Pyrazinyl-, Pyridazinyl-, Triazinyl-, Pyrimidinyl- Chinoxalinyl-, Chinolyl- oder Chinazolinylrest, $R_4$ Wasserstoff, $C_1$-$C_4$-Alkyl, vorzugsweise Methyl oder Isopropyl, oder Methoxy, $R_5$ Wasserstoff oder Methyl und X ein Sauerstoff- oder Schwefelatom bedeuten.

Besonders hervorzuheben sind ferner Verbindungen der Formel I, in denen das Strukturelement $-OR_3$ am Phenylring in meta- oder para-Stellung, vorzugsweise in para-Stellung, zum Barbitursäurecarbamoylrest steht.

Eine besonders erwähnenswerte Gruppe bilden Verbindungen der Formel I, in denen $R_1$ und $R_2$ Methyl, $R_3$ einen unsubstituierten oder ein- oder zweifach durch Substituenten der Gruppe Methyl, Isopropyl, Methoxy oder Chlor substituierten Pyrazinyl-, Pyrimidinyl-, Pyridazinyl-, Chinoxalinyl- oder Chinolylrest, $R_4$ und $R_5$ Wasserstoff und X ein Sauerstoff- oder Schwefelatom bedeuten.

Von herausragendem Interesse sind jedoch die nachfolgenden Verbindungsgruppen, deren Bedeutung hinsichtlich ihrer Wirksamkeit von a) nach f) zunimmt.

a) Verbindungen in denen $R_1$ $C_1$-$C_4$-Alkyl, $C_1$-$C_3$-Alkoxy, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten;

b) Verbindungen in denen $R_1$ $C_1$-$C_4$-Alkyl, Allyl, Cyclopropyl oder $C_1$-$C_3$-Alkoxy, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyrazinyl-, Pyrimidinyl-, Pyridazinyl-, Triazinyl-, Chinoxalinyl-oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten;

c) Verbindungen, in denen $R_1$ $C_1$-$C_4$-Alkyl, Allyl, Cyclopropyl oder Methoxy, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyrimidinyl-, Pyrazinyl- oder Pyridazinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten und der Molekülteil $-OR_3$ in meta- oder para-Stellung zum Stickstoffatom der Carbamoylgruppe steht;

d) Verbindungen, in denen $R_1$ Methyl oder Methoxy, $R_2$ Methyl, $R_3$ einen unsubstituierten oder ein- oder zweifach durch Substituenten der Gruppe $C_1$-$C_4$-Alkyl, Halogen-$C_1$-$C_4$-Alkyl, Halogen oder Phenyl substituierten, über ein Kohlenstoffatom gebundenen Pyrimidinylring, $R_4$ Wasserstoff, $C_1$-$C_4$-Alkyl oder $C_1$-$C_4$-Alkoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht;

e) Verbindungen, in denen $R_1$ Methyl, $R_2$ Methyl, $R_3$ einen ein- oder zweifach durch Substituenten der Gruppe Methyl, Trifluormethyl, Chlor oder Phenyl substituierten, über ein Kohlenstoffatom gebundenen Pyrimidinylring, $R_4$ Wasserstoff, Methyl, Isopropyl, Methoxy oder Aethoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht;

f) Verbindungen, in denen $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyrimidinylring, $R_4$ Wasserstoff, Methyl, Isopropyl oder Methoxy, $R_5$ Wasserstoff oder Methyl und X ein Sauerstoff- oder Schwefelatom bedeuten und der Molekülteil $-OR_3$ in para-Stellung zum Stickstoffatom der Carbamoylgruppe steht.

Bevorzugte Einzelverbindungen sind:

1,3-Dimethyl-5-[4-(6-chlor-1-oxy-pyridazin-3-yl-oxy)-phenylcarbamoyl]-barbitursäure,

1,3-Dimethyl-5-[4-(3-methylpyrazin-2-yl-oxy)-phenylcarbamoyl]-barbitursäure,

1,3-Dimethyl-5-[4-(3-chlorpyrazin-2-yl-oxy)-phenylcarbamoyl]-barbitursäure,

1,3-Dimethyl-5-[4-(3-methylpyrazin-2-yl-oxy)-phenylcarbamoyl]-2-thiobarbitursäure,

1,3-Dimethyl-5-[4-(6-trifluormethylpyrimidin-4-yl-oxy)-phenylcarbamoyl]-barbitursäure,

1,3-Dimethyl-5-[4-(4-trifluormethylpyrimidin-2-yl-oxy)-phenylcarbamoyl]-barbitursäure,

1,3-Dimethyl-5-[4-(2-triflormethylpyrimidin-4-yl-oxy)-phenylcarbamoyl]-2-thiobarbitursäure,

1,3-Dimethyl-5-[4-methoxy-3-(6-trifluormethylpyrimidin-4-yl-oxy)-phenylcarbamoyl]-2-thiobarbitursäure,

1,3-Dimethyl-5-[3-methoxy-4-(4-trifluormethylpyrimidin-2-yl-oxy)-phenylcarbamoyl]-2-thiobarbitursäure,

1,3-Dimethyl-5-[2-isopropyl-4-(6-trifluormethylpyrimidin-4-yl-oxy)-phenylcarbamoyl]-barbitursäure,

1,3-Dimethyl-5-[2,6-dimethyl-4-(2-trifluormethyl-pyrimidin-4-yl-oxy)-phenylcarbamoyl]-barbitursäure,

1,3-Dimethyl-5-[3-methoxy-4-(2-trifluormethylpyrimidin-4-yl-oxy)-phenylcarbamoyl]-barbitursäure,

1,3-Dimethyl-5-[4-(2-methyl-6-trifluormethylpyrimidin-4-yl-oxy)-phenylcarbamoyl]-barbitursäure,

1,3-Dimethyl-5-[2-isopropyl-4-(6-trifluormethylpyrimidin-4-yl-oxy)-phenylcarbamoyl]-2-thiobarbitursäure,

1,3-Dimethyl-5-[2,6-dimethyl-4-(2-trifluormethylpyrimidin-4-yl-oxy)-phenylcarbamoyl]-2-thiobarbitursäure,

1,3-Dimethyl-5-[4-(5-chlorpyrimidin-2-yl-oxy)-phenylcarbamoyl]-barbitursäure,

1,3-Dimethyl-5-[3-methoxy-4-(5-methyl-4-trifluormethylpyrimidin-2-yl-oxy)-phenylcarbamoyl]-barbitursäure,

1,3-Dimethyl-5-[4-(5-phenyl-4-trifluormethylpyrimidin-2-yl-oxy)-phenylcarbamoyl]-barbitursäure,
1,3-Dimethyl-5-[4-(4-methyl-6-trifluormethylpyrimidin-2-yl-oxy)-phenylcarbamoyl]-barbitursäure,
1,3-Dimethyl-5-[4-(4-methyl-6-trifluormethylpyrimidin-2-yl-oxy)-phenylcarbamoyl]-2-thiobarbitursäure,
1,3-Dimethyl-5-[4-(6-methyl-2-trifluormethylpyrimidin-4-yl-oxy)-phenylcarbamoyl]-barbitursäure und
1,3-Dimethyl-5-[4-(2-Trifluormethylpyrimidin-4-yl-oxy)-phenylcarbamoyl]-barbitursäure

Es wurde nun gefunden, dass die erfindungsgemässen neuen Verbindungen der Formel I überraschenderweise ein sehr günstiges Wirkungsspektrum gegen tierparasitäre Helminthen besitzen, vor allem gegen solche, welche in Warmblütern, insbesondere Säugetieren, parasitieren. Die Verbindungen der Formel I sind gegen Nematoden sowie Cestoden und Trematoden mit gutem Erfolg anwendbar. Dabei zeichnen sie sich vor allem dadurch aus, dass sie auch gegen benzimidazol-resistente, insbesondere gegen thiabendazol-resistente Helminthen voll wirksam sind ("thiabendazol" bezeichnet den Wirkstoff 2-(Thiazol-4-yl)-benzimidazol).

Die Verbindungen der Formel I werden hergestellt, indem man
a) eine Verbindung der Formel II

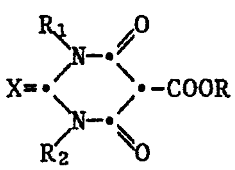

(II)

worin $R_1$, $R_2$ und X die für Formel I angegebenen Bedeutungen haben und R $C_1$-$C_5$-Alkyl oder unsubstituiertes oder durch Nitro substituiertes Phenyl bedeutet, mit einer Verbindung der Formel III

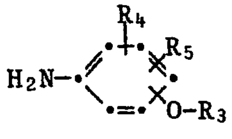

(III)

worin $R_3$, $R_4$ und $R_5$ die für Formel I angegebenen Bedeutungen haben, umsetzt, oder

b) eine Verbindung der Formel IV

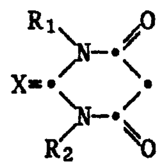

(IV)

worin $R_1$, $R_2$ und X die für Formel I angegebenen Bedeutungen haben, mit einer Verbindung der Formel V

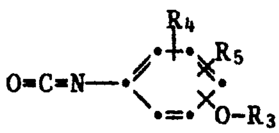

(V)

worin $R_3$, $R_4$ und $R_5$ die für Formel I angegebenen Bedeutungen haben, umsetzt, oder

c) eine Verbindung der Formel IV, worin $R_1$, $R_2$ und X die für Formel I angegebenen Bedeutungen haben, mit einer Verbindung der Formel VI

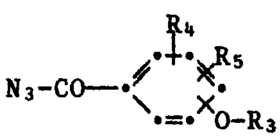

(VI)

worin $R_3$, $R_4$ und $R_5$ die für Formel I angegebenen Bedeutungen haben, umsetzt, oder

d) eine Verbindung der Formel VII

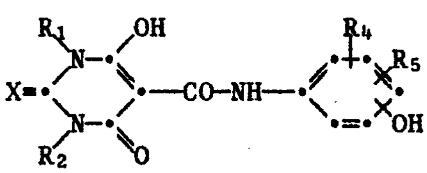

(VII)

worin $R_1$, $R_2$, $R_4$, $R_5$ und X die für Formel I angegebenen Bedeutungen haben, mit einer Verbindung der Formel VIII

$Q-R_3$ (VIII)

worin Q für eine übliche Abgangsgruppe steht und $R_3$ die für Formel I angegebene Bedeutung hat, in Gegenwart einer Base umsetzt, oder

e) zur Herstellung von Verbindungen der Formel I, in denen mindestens einer der Substituenten $Z_1$, $Z_2$, $Z_3$, $Z_4$ und $Z_5$ für Wasserstoff, $C_1$-$C_4$-Alkoxy, $C_1$-$C_4$-Alkylthio, $C_1$-$C_3$-Alkylamino oder Di-($C_1$-$C_3$-alkyl)-amino steht, eine Verbindung der Formel IX

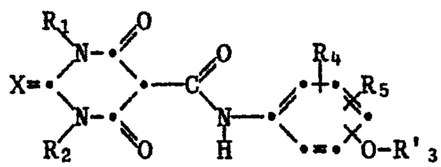

(IX)

worin $R_1$, $R_2$, $R_4$, $R_5$ und X die für Formel I angegebenen Bedeutungen haben und $R'_3$ für einen heteroaromatischen 6-gliedrigen Ring, welcher 2 bis 3 Stickstoffatome enthält oder einen benzokondensierten, heteroaromatischen Ring, welcher 1 bis 3 Stickstoffatome enthält, wobei der Ring oder der heterocyclische Teil des Ringsystems durch Halogen, bevorzugt Chlor, oder Methylsulfonyl substituiert ist, steht, mit einer Verbindung der Formel X

Z-H (X)

worin Z für $C_1$-$C_4$-Alkoxy, $C_1$-$C_4$-Alkylthio, $C_1$-$C_3$-Alkylamino oder Di-($C_1$-$C_3$-alkyl)-amino steht, umsetzt.

Die Herstellungsvarianten (a) und (c) werden bei Reaktionstemperaturen zwischen 50° und 250°C, vorzugweise 70° bis 220°C, durchgeführt. Variante (b) erfordert Reaktionstemperaturen zwischen 0° und 220°C, insbesondere 0° bis 200°C. Die Variante (d) findet bei Reaktionstemperaturen von 50° bis 250°C, vorzugsweise 80° bis 150°C, in reaktionsinerten Lösungs- oder Verdünnungsmitteln statt. Die Reaktionen (a), (b) und (c) können bei normalem oder erhöhtem Druck und in Abwesenheit oder vorzugsweise in Gegenwart reaktionsinerter Lösungs- oder Verdünnungsmittel durchgeführt werden, wobei in manchen Fällen vorteilhafterweise mit einer Base gearbeitet wird.

Die Herstellungsvariante e) lässt sich am vorteilhaftesten auf folgende Weise durchführen: $e_1$) wenn Z für Wasserstoff steht, durch Umsetzung einer Verbindung der Formel IX mit einer Verbindung der Formel X in Gegenwart eines Katalysators wie beispielsweise Raney-Nickel oder Palladium auf Aktivkohle; $e_2$) wenn Z für $C_1$-$C_4$-Alkoxy oder $C_1$-$C_4$-Alkylthio steht, durch Umsetzung einer Verbindung der Formel IX mit einer Verbindung der Formel X in Gegenwart einer Base oder nach Ueberführung von Z-H in üblicher Weise in ein Salz $Z-M^{\oplus}$, worin $M^{\oplus}$ für ein geeignetes Kation wie beispielsweise $Na^{\oplus}$ oder $K^{\oplus}$ steht, in einem inerten Lösungsmittel und bei einer Temperatur von 20 bis 200°C vorzugsweise 50 bis 150°C; oder $e_3$) wenn Z für $C_1$-$C_3$-Alkylamino oder Di-($C_1$-$C_3$-Alkyl)-amino steht, in Gegenwart einer Base oder unter Anwendung von Z-H im Ueberschuss und bei einer Temperatur wie vorstehend unter $e_2$) angegeben.

Die Herstellung der erfindungsgemässen Salze von Verbindungen der Formel I erfolgt durch übliche Neutralisation der freien Säure mit einer Base, insbesondere einer physiologisch verträglichen Base. Vorzugsweise genannt seien Alkalisalze wie Natrium-, Kalium- oder Lithiumsalze sowie Ammoniumsalze und Trialkylaminsalze wie z.B. das bevorzugte Triethylaminsalz. Die Neutralisation wird in einem reaktionsinerten polaren Lösungsmittel, z.B. einem Alkanol, Ester oder einer etherartigen Verbindung, durchgeführt.

N-oxyde von Verbindungen der Formel I lassen sich auf an sich bekannte Weise herstellen, beispielsweise durch Oxydation von erhaltenen Verbindungen der Formel I mit Peroxyden.

Die für die Herstellung von Verbindungen der Formel I gemäss Verfahrensvarianten a) und d) besonders entwickelten Zwischenprodukte der Formeln III und VII sind neu und bilden ebenso wie die nachfolgend beschriebenen Herstellungsverfahren einen Gegenstand der vorliegenden Erfindung.

Die Herstellung von Verbindungen der Formel VII erfolgt analog bekannten Verfahren durch Umsetzung einer Verbindung der Formel II, worin $R_1$, $R_2$ und X die für Formel I angegebenen, insbesondere die in Tabelle 1 aufgeführten Bedeutungen haben und R $C_1$-$C_5$-Alkyl oder unsubstituiertes oder durch Nitro substituiertes Phenyl bedeutet, mit einer Verbindung der Formel XI

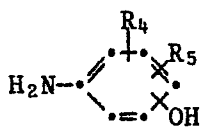

(XI)

worin $R_4$ und $R_5$ die für Formel I angegebenen, insbesondere die in Tabelle 1 aufgeführten Bedeutungen haben, umsetzt. Die Umsetzung erfolgt unter den gleichen Bedingungen, wie sie vorstehend unter Verfahrensvariante a) für die Umsetzung von Verbindungen der Formel II mit Verbindungen der Formel III angegeben sind.

Die Herstellung von Verbindungen der Formel III erfolgt analog Verfahrensvariante d) durch Umsetzung einer Verbindung der Formel XI, worin $R_4$ und $R_5$ die für Formel I angegebenen, insbesondere die in Tabelle 1 aufgeführten Bedeutungen haben, mit einer Verbindung der Formel VIII, worin $R_3$ die für Formel I angegebenen, insbesondere die in Tabelle 1 aufgeführten Bedeutungen hat und Q für eine übliche Abgangsgruppe steht. Die Umsetzung erfolgt unter den gleichen Bedingungen, wie sie vorstehend unter Verfahrensvariante d) für die Umsetzung von Verbindungen der Formel VII mit Verbindungen der Formel VIII angegeben sind.

Q steht in Formel VIII entweder für eine der üblichen Abgangsgruppen, z.B. Halogen, insbesondere Chlor, Brom oder Jod; für eine Sulfonyloxygruppe, insbesondere Benzolsulfonyloxy, Paratosyloxy oder Niederalkylsulfonyloxy, bevorzugt Mesyloxy; oder für eine Acyloxygruppe wie Trifluoracetyloxy. Q steht auch für eine Hydroxygruppe oder gemäss "Synthesis" 1979, p-561-569, für den Rest

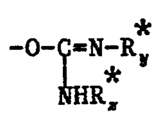

worin $R_y^*$ und $R_x^*$ als organische Reste beispielsweise Isopropyl oder p-Tolyl repräsentieren. Die Reste können jedoch auch für andere Niederalkylreste oder für gegebenenfalls substituiertes Phenyl stehen, wobei als Substituenten beispielsweise diejenigen in Betracht kommen, die unter $Z_4$ und $Z_5$ an vorgängiger Stelle aufgeführt sind.

Als Vertreter von Verbindungen der Formel III sind beispielsweise die folgenden zu nennen:
4-(3-Methylpyrazin-2-yl-oxy)-anilin, Smp. 108-109°C;
4-(3-Chlorpyrazin-2-yl-oxy)-anilin, Smp. 130-131°C;
4-(6-Chlorchinoxalin-2-yl-oxy)-anilin, Smp. 107-112°C;
4-(Pyrimidin-2-yl-oxy)-anilin, Smp. 125-129°C;
4-(4-Chlorpyrimidin-2-yl-oxy)-anilin, Oel;
4-(4,6-Dimethylpyrimidin-2-yl-oxy)-anilin, Smp. 92-96°C;
4-(6-Chlorpyrimidin-4-yl-oxy)-anilin, Oel;
4-(2-Methylthio-6-methyl-pyrimidin-4-yl-oxy)-anilin, Oel;
4-(2-tert.-Butyl-6-chlor-pyrimidin-4-yl-oxy)-anilin, Oel;
4-(Chinolin-2-yl-oxy)-anilin;
3-(6-Chlorchinoxalin-2-yl-oxy)-anilin;
4-(3-Methoxychinoxalin-2-yl-oxy)-anilin;
3-(Chinolin-2-yl-oxy)-anilin;
2,6-Dimethyl-4-(pyrazin-2-yl-oxy)-anilin;
3-(6-Chlorpyridazin-3-yl-oxy)-anilin;
4-(4,6-Dimethoxy-1,3,5-triazin-2-yl-oxy)-anilin;
4-(4,6-Dimethylthio-1,3,5-triazin-2-yl-oxy)-anilin.

Für die Herstellung der erfindungsgemässen Wirksubstanzen geeignete Lösungs- oder Verdünnungsmittel sind z.B. Ether und etherartige Verbindungen wie Dialkylether (Diethylether, Diisopropylether, tert.-Butylmethylether usw.), Anisol, Dioxan, Tetrahydrofuran; aliphatische und aromatische Kohlenwasserstoffe wie Benzol, Toluol, Petrolether; halogenierte Kohlenwasserstoffe wie Chlorbenzol, Methylenchlorid, Chloroform, Ethylenchlorid, Tetrachlorkohlenstoff, Tetrachlorethylen; Nitrile wie Acetonitril, Propionitril; N,N-dialkylierte Amide wie Dimethylformamid; Dimethylsulfoxid; Ketone wie

Aceton, Diethylketon, Methylethylketon sowie insbesondere für Verfahrensvariante (d) Wasser und Alkohole wie z.B. Methanol, Ethanol, Isopropanol oder Butanol; und ganz allgemein Gemische solcher Lösungsmittel untereinander.

Als Basen kommen organische und anorganische Basen in Betracht; z.B. vorzugsweise tertiäre Amine wie Trialkylamine (Trimethylamin, Triethylamin, Tripropylamin usw.), Pyridin und Pyridinbasen (z.B. 4-Dimethylaminopyridin, 4-Pyrrolidylaminopyridin usw.), Picoline und Lutidine sowie Oxide, Hydroxide, Carbonate und Hydrogencarbonate von Alkali- und Erdalkalimetallen (z.B. CaO, BaO, NaOH, KOH, $Ca(OH)_2$, $KHCO_3$, $NaHCO_3$, $Ca(HCO_3)_2$, $K_2CO_3$, $Na_2CO_3$ usw.), ferner Acetate wie z.B. $CH_3COONa$ oder $CH_3COOK$. Darüber hinaus eignen sich als Basen auch Alkalialkoholate wie z.B. Natriumethylat, Natriumpropylat, Kalium-tert.-butylat oder Natriummethylat. Die Base wird für die Verfahrensvarianten (a), (b) und (c) vorteilhafterweise in 10 bis 100 % der äquimolaren Menge und für die Verfahrensvariante (d) in 200 % der äquimolaren Menge, bezogen auf die Reaktanden, zugesetzt.

In manchen Fällen kann es von Vorteil sein, wenn die Reaktion unter Schutzgasatmosphäre durchgeführt wird. Geeignete Schutzgase sind z.B. Stickstoff, Helium, Argon oder Kohlendioxid.

Verbindungen der Formel I, welche in Salzform vorliegen, lassen sich auf an sich bekannte Weise in die freie Form überführen.

Die in den Herstellungsvarianten (a), (b), (c), (d) und (e) genannten Ausgangsstoffe sind - mit Ausnahme der in der Verfahrensvariante (a) eingesetzten Verbindungen der Formel III und der in der Verfahrensvariante (d) eingesetzten Verbindungen der Formel VII - bekannt (siehe z.B. Chem. Ber. 54, 1038 [1921]) oder können in analoger Weise wie die bekannten Substanzen hergestellt werden.

Das beschriebene Herstellungsverfahren ist einschliesslich aller Varianten (a), (b), (c), (d) und (e) ein Bestandteil vorliegender Erfindung.

Die erfindungsgemässen Wirkstoffe der Formel I können in unterschiedlichen tautomeren Formen vorliegen, nämlich in der Keto- oder Enolform oder in einem Gemisch aus Keto- und Enolform. Diese Erfindung betrifft sowohl die einzelnen Tautomeren als auch deren Gemische, aber auch die Salze jeder dieser Formen und deren Herstellung. Dasselbe gilt auch für die N-oxyde der Wirkstoffe der Formel I.

Die Erfindung schliesst auch ein Verfahren zum prophylaktischen Schutz von Tieren gegen parasitäre Helminthen ein, das dadurch gekennzeichnet ist, dass man die Wirkstoffe der Formel I bzw. die Wirkstofformulierungen als Zusatz zum Futter oder zu den Tränken oder auch in fester oder flüssiger Form oral, durch Injektion oder mittels der Pour-on-Methode den Tieren appliziert.

In den erfindungsgemässen Verfahren zur Helminthenbekämpfung bzw. den erfindungsgemässen Helminthenbekämpfungsmitteln können die Wirkstoffe der Formel I in allen tautomeren Formen, deren Mischungen oder in Form ihrer Salze eingesetzt werden.

Unter den bei Warmblütern vorkommenden Endoparasiten verursachen namentlich die Helminthen grosse Schäden. So können von diesen Parasiten befallene Tiere nicht nur ein verlangsamtes Wachstum, sondern erhebliche physiologische Beeinträchtigungen aufweisen, die sogar zur Mortalität führen können. Daher ist es von grosser Bedeutung, therapeutische Mittel zu entwickeln, die sich zur Bekämpfung von Helminthen und deren Entwicklungsstadien sowie zur Vorbeugung gegen den Befall durch diese Parasiten eignen. Besonders gefährliche Wurmkrankheiten sind solche, die durch im Magen-Darmtrakt und anderen Organen parasitierende Nematoden, Cestoden und Trematoden hervorgerufen werden und vor allem bei Wiederkäuern, wie Schafen, Rindern und Ziegen sowie Pferden, Schweinen, Rotwild, Hunden, Katzen und Geflügel auftreten.

Die durch Helminthiasen verursachten Schäden können bei chronischem und vor allem bei epidemischem Auftreten der Wurmerkrankungen in Viehherden beträchtlich sein. Sie äussern sich unter anderem in Produktivitäts-Verminderungen, geschwächter Widerstandskraft und erhöhter Mortalität. Bekämpfung und Vorbeugung von Helminthiasen gelten deshalb als vordringliche Aufgabe, um derartige vor allem volkswirtschaftlich ins Gewicht fallende Schäden zu vermeiden oder zu mindern.

In der vorliegenden Beschreibung werden unter dem Begriff "Helminthen" insbesondere parasitäre Würmer verstanden, die zu den Phyla Plathelminthes (Cestoden, Trematoden) und Nemathelminthes (Nematoden und Verwandte) gehören, also Bandwürmer, Saugwürmer und Rundwürmer des Gastrointestinal-Traktes und anderer Organe (z.B. Leber, Lunge, Niere, Lymphgefässe, Blut etc.). Es sind zwar eine Reihe von Stoffen mit anthelmintischer Wirkung bekannt, die für die Bekämpfung der verschiedenen Helminthen-species vorgeschlagen wurden. Diese vermögen jedoch nicht voll zu befriedigen, sei es, dass bei verträglicher Dosierung eine Ausschöpfung ihres Wirkungsspektrums nicht möglich ist, oder dass sie in therapeutisch wirksamen Dosen unerwünschte Nebenwirkungen oder Eigenschaften zeigen. In diesem Zusammenhang spielt auch die heute vermehrt auftretende Resistenz gegen bestimmte Stoffklassen eine immer bedeutendere Rolle. Das beispielsweise in der Literatur beschriebene "Albendazol" (British Pat. No. 1464326; Am. J. Vet. Res. 38, 1425-1426 (1977); Am. J. Vet. Res. 37, 1515-1516 (1976); Am. J. Vet. Res. 38, 807-808 (1977); Am. J. Vet. Res. 38, 1247-1248 (1977)) besitzt zwar ein begrenztes anthelmintisches Wirkungsspektrum bei Wiederkäuern, seine Wirkung gegen Benzimidazol-resistente Nematoden und adulte Leberegel ist jedoch unzureichend, da vor allem die pathologisch wichtigen unreifen Wanderformen der letzteren bei den für das Wirtstier verträglichen Dosierungen nicht angegriffen werden.

Ueberraschenderweise wurde nun festgestellt, dass die Wirkstoffe der Formel I nicht nur - wie bereits erwähnt - eine intensive anthelmintische Wirksamkeit mit breitem Wirkungsspektrum gegen Nematoden, Cestoden und Trematoden, sondern darüber hinaus zusätzlich eine günstige Warmblütertoxizität besitzen.

Die erfindungsgemässen neuen Wirkstoffe der Formel I sind beispielsweise zur Bekämpfung parasitärer Nematoden der Ordnungen (nach K.I. Skrajabin)

Rhabditida
Ascaridida
Spirurida
Trichocephalida

oder zur Bekämpfung von Cestoden der Ordnungen (nach Wardle & McLeod)

Cyclophyllidae
Pseudophyllidae

oder zur Bekämpfung von Trematoden der Ordnung

Digenea

bei Haus- und Nutztieren wie Rindern, Schafen, Ziegen, Pferden, Schweinen, Katzen, Hunden und Geflügel geeignet. Sie können den Tieren sowohl als Einzeldosis als auch wiederholt verabreicht werden, wobei die einzelnen Gaben je nach Tierart vorzugsweise zwischen 1 und 500 mg pro kg Körpergewicht betragen. Durch eine protrahierte Verabreichung wird in manchen Fällen eine bessere Wirkung erzielt oder man kann mit geringeren Gesamtdosen auskommen.

Die erfindungsgemäsen Mittel werden hergestellt, indem die Wirkstoffe der Formel I mit flüssigen und/oder festen Formulierungshilfsstoffen durch schrittweises Vermischen und/oder Vermahlen derart in Kontakt gebracht werden, dass eine anwendungskonforme optimale Entfaltung der anthelmintischen Aktivität der Formulierung erzielt wird.

Die Formulierungsschritte können durch Kneten, Granulieren (Granulate) und gegebenenfalls Pressen (Pellets) ergänzt werden.

Als Formulierungshilfsstoffe dienen beispielsweise feste Trägerstoffe, Lösungsmittel und gegebenenfalls oberflächenaktive Stoffe (Tenside).

Zur Bereitung der erfindungsgemässen Mittel werden folgende Formulierungshilfsstoffe verwendet:
Feste Trägerstoffe wie z.B. Kaolin, Talkum, Bentonit, Kochsalz, Calciumphosphat, Kohlenhydrate, Cellulosepulver, Baumwollsaatmehl, Polyäthylenglykoläther, gegebenenfalls Bindemittel wie z.B. Gela tine, lösliche Cellulosederivate, gewünschtenfalls unter Zusatz von oberflächenaktiven Stoffen wie ionischen oder nicht-ionischen Dispersionsmitteln; ferner natürliche Gesteinsmehle wie Calcit, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüber hinaus kann eine Vielzahl von vorgranulierten Materialien anorganischer oder organischer Natur wie insbesondere Dolomit oder zerkleinertes Pflanzenmaterial verwendet werden.

Als Lösungsmittel kommen in Frage: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat; aliphatische Kohlenwasserstoffe wie z.B. Cyclohexan oder Paraffine, Alkohole und Glykole sowie deren Ether und Ester, wie z.B. Ethanol, Ethylenglykol, Ethylenglykolmonomethyl- oder ethylether, Ketone wie z.B. Cyclohexanon, stark polare Lösungsmittel wie z.B. N-Methyl-2-pyrrolidon, Dimethylsulfoxid oder Dimethylformamid, sowie gegebenenfalls epoxydierte Pflanzenöle wie z.B. epoxydiertes Kokosnussöl oder Sojaöl und Wasser.

Als oberflächenaktive Verbindungen kommen je nach Art des zu formulierenden Wirkstoffes der Formel I nicht-ionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt.

Häufig werden sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxid-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure oder eines Naphthalinsulfonsäure-Formaldehydkondensationsproduktes.

Ferner können auch entsprechende Phosphate, wie z.B. Salze des Phosphorsäureesters eines p-Nonylphenol-(4-14)-Ethylenoxyd-Adduktes oder Phospholipide als Formulierungshilfsstoffe Anwendung finden.

Als nicht-ionische Tenside kommen in erster Linie Polyglykoletherderivate von aliphatischen oder cycloaliphatischen Alkoholen, gesättigten oder ungesättigten Fettsäuren und Alkyphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nicht-ionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nicht-ionischer Tenside seien Nonylphenolpolyethoxyethanole, Ricinusölpolyglykolether, Polypropylen-Polyethylenoxydaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood New Jersey, 1981;
Stache, H., "Tensid-Taschenbuch",
Carl Hanser Verlag, München/Wien, 1981

Als Bindemittel für Tabletten und Boli kommen chemisch abgewandelte, in Wasser oder Alkohol lösliche, polymere Naturstoffe in Frage, wie Stärke-, Cellulose- oder Proteinderivate (z.B. Methylcellulose, Carboxymethylcellulose, Ethylhydroxyethylcellulose, Proteine wie Zein, Gelatine und dergleichen) sowie synthetische Polymere wie z.B. Polyvinylalkohol, Polyvinylpyrrolidon etc.. Ferner sind in Tabletten Füllstoffe, (z.B. Stärke, mikrokristalline Cellulose, Zucker, Milchzucker etc.), Gleitmittel und Sprengmittel enthalten.

Liegen die anthelmintischen Mittel in Form von Futterkonzentraten vor, so dienen als Trägerstoffe z.B. Leistungsfutter, Futtergetreide oder Proteinkonzentrate. Solche Futterkonzentrate oder -mittel können ausser den Wirkstoffen noch Zusatzstoffe, Vitamine, Antibiotika, Chemotherapeutika, oder Pestizide, vornehmlich Bakteriostaika, Fungistatika, Coccidiostatika, oder auch Hormonpräparate, Stoffe mit anaboler Wirkung oder das Wachstum begünstigende, die Fleischqualität von Schlachttieren beeinflussende oder in anderer Weise für den Organismus nützliche Stoffe enthalten. Werden die Mittel oder die darin enthaltenen Wirkstoffe der Formel I direkt dem Futter oder den Viehtränken zugesetzt, so enthält das Fertigfutter oder die Fertigtränke die Wirkstoffe vorzugsweise in einer Konzentration von etwa 0,0005 bis 0,02 Gewichtsprozent (5-200 ppm).

Die Applikation der erfindungsgemässen Mittel an die zu behandelnden Tiere kann peroral, parenteral, subcutan oder topikal durchgeführt werden, wobei die Mittel in Form von Lösungen, Emulsionen, Suspensionen (Drenchs), Pulvern, Tabletten, Boli und Kapseln vorliegen.

Die erfindungsgemässen anthelmintischen Mittel enthalten in der Regel 0,1 bis 99 Gew.%, insbesondere 0,1 bis 95 Gew.-%, Wirkstoff der Formel I, und 99,9 bis 1 Gew.-%, insbesondere 99,9 bis 5 Gew.-%, eines festen oder flüssigen Zusatzstoffes, darunter 0 bis 25 Gew.-%, insbesondere 0,1 bis 25 Gew.-%, eines Tensides.

Während als Handelsware eher konzentrierte Mittel bevorzugt werden, verwendet der Endverbraucher in der Regel verdünnte Mittel.

Solche Mittel können noch weitere Zusätze wie Stabilisatoren, Entschäumer, Viskositätsregulatoren, Bindemittel, Haftmittel sowie andere Wirkstoffe zur Erzielung spezieller Effekte enthalten.

Derartige vom Endverbraucher verwendete anthelmintische Mittel sind ebenfalls ein Bestandteil der vorliegenden Erfindung.

Die nachfolgenden Beispiele dienen zur näheren Erläuterung der Erfindung, ohne dieselbe einzuschränken.

## 1. Herstellungsbeispiele

### 1.1. 1,3-Dimethyl-5-[4-(2-isopropyl-6-methyl-pyrimidin-4-yl-oxy)-phenylcarbamoyl]-barbitursäure

3,0 g (0,013 Mol) 1,3-Dimethyl-5-ethoxycarbonyl-barbitursäure und 3,2 g (0,013 Mol) 4-(2-Isopropyl-6-methyl-pyrimidin-4-yl-oxy)-anilin werden in 30 ml Toluol suspendiert und unter Schutzgasstrom (Stickstoff) 18 Stunden auf Rückflusstemperatur erhitzt. Nach Abkühlen wird der Niederschlag abfiltriert, mit Alkohol nachgewaschen und getrocknet.
Ausbeute: 4,4 g (80 % der Theorie), Smp. 147-149°C.

1.2. 1,3-Dimethyl-5-[4-(3,5-dichlor-pyrimidin-4-yl-oxy)-phenylcarbamoyl]-barbitursäure

7,8 g (0,050 Mol) 1,3-Dimethyl-barbitursäure und 13,5 g (0,050 Mol) 4-(2-Isopropyl-6-methyl-pyrimidin-4-yl-oxy)-phenylisocyanat werden in 50 ml Xylol suspendiert und tropfenweise mit 1 g (0,010 Mol) Triethylamin versetzt. Die Temperatur steigt auf 45° bis 50°C. Nach weiterer Zugabe von 50 ml Xylol wird das Gemisch 18 Stunden bei dieser Temperatur gerührt. Dann wird ein Drittel des Xylols abdestilliert. Nach Abkühlung wird der Niederschlag abfiltriert, mit Xylol nachgewaschen, mehrmals in Wasser suspendiert und anschliessend gründlich mit Wasser gewaschen und getrocknet.

1.3. 1,3-Dimethyl-5-[4-(2-isopropyl-6-methyl-pyrimidin-4-yl-oxy)-phenylcarbamoyl]-barbitursäure

Zu einer Lösung von 3,85 g (0,01 Mol) 4-(2-Isopropyl-6-methyl-pyrimidin-4-yl-oxy)-benzoylazid in 50 ml Toluol werden 1,56 g (0,01 Mol) 1,3-Dimethylbarbitursäure gegeben. Anschliessend wird eine Lösung von 0,02 g (0,002 Mol) Triäthylamin in 5 ml Toluol bei Raumtemperatur zugetropft. Das Gemisch wird dann 1 Stunde bei 50°C gerührt und danach die Temperatur stufenweise um jeweils 20°C erhöht bis die Rückflusstemperatur erreicht ist. Die Rückflusstemperatur wird bis zur Beendigung der Stickstoffentwicklung aufrechterhalten. Nach Abkühlung wird der ausgefallene Niederschlag abfiltriert, mit Aethanol nachgewaschen, mehrmals in 1 N HCl suspendiert und anschliessend gründlich mit Wasser gewaschen und getrocknet.
Ausbeute: 3 g (60 % der Theorie), Smp. 147-149°C.

Das als Vorstufenpräparat eingesetzte 4-(2-Isopropyl-6-methyl-pyrimidin-4-yl-oxy)-benzoylazid wird wie folgt hergestellt:
6,4 g (0,024 Mol) 4-(2-Isopropyl-6-methyl-pyrimidin-4-yl-oxy)-benzoesäure werden in 40 ml Aceton bei 0°C mit 3,4 g (0,031 Mol) Chlorameisensäureäthylester in Gegenwart von 2,9 g (0,028 Mol) Triäthylamin versetzt und weitere 2,4 g (0,036 Mol) Natriumazid in 8 ml Wasser dazu gegeben. Nach dreistündigem Rühren bei 0°C wird das

Gemisch in 100 ml Wasser gegossen und mit 60 ml Toluol extrahiert. Die Toluolphase wird abgetrennt, über Natriumsulfat bei 0°C getrocknet und nach Filtration für die oben beschriebene Reaktion verwendet.

1.4. 1,3-Dimethyl-5-[4-(2-isopropyl-6-methyl-pyrimidin-4-yl-oxy)-phenylcarbamoyl]-barbitursäure

Eine Suspension von 7,8 g (0,050 Mol) 1,3-Dimethyl-barbitursäure und 19,2 g (0,050 Mol) 4-(2-Isopropyl-6-methyl-pyrimidin-4-yl-oxy)-benzoylazid in 50 ml Xylol werden tropfenweise mit 1 g (0,010 Mol) Triethylamin versetzt. Die Temperatur steigt auf 45° bis 50°C. Nach weiterer Zugabe von 50 ml Xylol wird das Gemisch 18 Stunden bei dieser Temperatur gerührt. Dann wird ein Drittel des Xylols abdestilliert. Nach Abkühlung wird der Niederschlag abfiltriert, mit Xylol nachgewaschen, mehrmals in Wasser suspendiert und anschliessend gründlich mit Wasser gewaschen und getrocknet.
Ausbeute: 15 g (60 % der Theorie), Smp. 147-149°C.

1.5 1,3-Dimethyl-5-[4-(3-methyl-pyrazin-2-yl-oxy)-phenylcarbamoyl]-2-thiobarbitursäure

2,5 g (10 mMol) 1,3-Dimethyl-5-ethoxycarbonyl-2-thiobarbitursäure, 2,0 g (10 mMol) 4-(3-Methyl-pyrazin-2-yl-oxy)anilin, und 30 ml Aethanol werden gemischt und unter Rühren 20 Stunden auf 60°C gehalten. Dann lässt man abkühlen, filtriert das gebildete Kristallisat ab, wäscht mit Aethanol nach und trocknet im Vakuum bei 80°C.
Ausbeute: 1,4 g; Smp. 167-169°C.

1.6. 1,3-Dimethyl-5-[4-(6-methoxypyridazin-3-yl-oxy)-phenylcarbamoyl]-barbitursäure

8,7 g (0,03 Mol) 1,3-Dimethyl-5-(4-hydroxyphenylcarbamoyl)barbitursäure werden in 70 ml Toluol und 50 ml Dimethylsulfoxid mit 4,0 g (0,06 Mol) 85 %igem Kaliumhydroxid versetzt. Das Gemisch wird mit Wasserabscheider bei Rückflusstemperatur entwässert. Nach Abdestillieren des Toluols werden 4,3 g (0,03 Mol) 3-Chlor-6-methoxypyridazin zugegeben. Die Badtemperatur wird allmählich erhöht, bis eine Temperatur zwischen 120 und 140°C erreicht ist, welche 2 Stunden beibehalten wird. Dann wird das Gemisch auf 80°C abgekühlt, mit verdünnter Salzsäure neutralisiert, auf Raumtemperatur abgekühlt und mit Wasser verdünnt. Der Niederschlag wird abfiltriert, mit Wasser gewaschen und getrocknet.
Ausbeute: 7 g (60 % der Theorie), Smp. 237°C.

1.7 <u>1,3-Dimethyl-5-[4-(6-methoxypyridazin-3-yl-oxy)-phenylcarbamoyl]-barbitursäure</u>

1,0 g (0,0025 Mol) 1,3-Dimethyl-5-[4-(6-chlorpyridazin-3-yl-oxy)-phenylcarbamoyl]-barbitursäure wird in 20 ml Methanol mit der aequimolaren Menge Natriummethylat bei Raumtemperatur versetzt. Das Gemisch wird 5 Stunden lang unter Rückfluss erhitzt, dann abgekühlt, mit Wasser verdünnt, abfiltriert, mit Wasser gewaschen und getrocknet.
Ausbeute: 0,7 g (70 % der Theorie), Smp. 237°C

Herstellung des Ausgangsprodukts 1,3-Dimethyl-5-[4-(6-chlorpyridazin-3-yl-oxy)-phenylcarbamoyl]-barbitursäure:

7,3 g (0,032 Mol) 1,3-Dimethyl-5-ethoxycarbonyl-barbitursäure und 7,1 g (0,032 Mol) 4-(6-Chlorpyridazin-3-yl-oxy)-anilin werden in 100 ml Toluol suspendiert und unter Schutzgasstrom (Stickstoff) 3 Stunden auf Rückflusstemperatur erhitzt, wobei Ethanol entweicht. Nach Abkühlen auf 80°C wird das Gemisch mit 80 ml Aethanol verdünnt, abgekühlt, abfiltriert und in Chloroform/Aethanol umkristallisiert.
Ausbeute: 4,0 g (30 % der Theorie), Smp. 265-267°C.

1.8 1,3-Dimethyl-5-[4-(chinolin-2-yl-oxy)-phenylcarbamoyl]-barbitursäure

6,1 g 4-(Chinolin-2-yl-oxy)-anilin und 5,8 g 1,3-Dimethyl-5-ethoxycarbonylbarbitursäure werden in 50 ml Toluol unter Rückfluss erhitzt. Wenn die Temperatur bei 80°C liegt, formt sich ein dicker Kristallbrei, welcher bei 120°C wieder eine rührfähige Konsistenz aufweist. Nach 3-stündigem Rühren wird das Gemisch abgekühlt und die Kristalle werden abgenutscht. Ausbeute: 9,9 g in Form beigefarbener Kristalle; Smp. 220-222°C.

Analog einer der vorstehend beschriebenen Methoden lassen sich auch die folgenden, in der Tabelle 1 zusammen mit den Verbindungen der vorstehenden Beispiele 1.1 bis 1.8 aufgeführten Verbindungen der Formel I herstellen:

- 27 -

Tabelle 1: Verbindungen der Formel

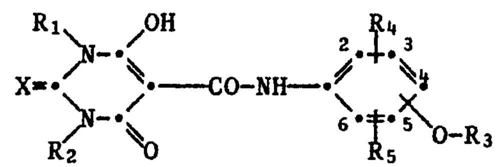

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 1 | $CH_3$ | $CH_3$ | H | H | O | 4 | 3-Methylpyrazin-2-yl | Smp. 185-187 |
| 2 | $CH_3$ | $CH_3$ | H | H | O | 4 | 3-Chlorpyrazin-2-yl | Smp. 206-208 |
| 3 | $CH_3$ | $CH_3$ | H | H | O | 4 | 6-Chlorchinoxalin-2-yl | Smp. 228-231 |
| 4 | $CH_3$ | $CH_3$ | H | H | O | 4 | Pyrimidin-2-yl | Smp. 197 |
| 5 | $CH_3$ | $CH_3$ | H | H | O | 4 | 4,6-Dimethylpyrimidin-2-yl | Smp. 199-203 |
| 6 | $CH_3$ | $CH_3$ | H | H | O | 4 | 4-Chlorpyrimidin-2-yl | Smp. 226-228 |
| 7 | $CH_3$ | $CH_3$ | H | H | O | 4 | 6-Chlorpyrimidin-4-yl | Smp. 194-196 |
| 8 | $CH_3$ | $CH_3$ | H | H | O | 4 | Chinolin-2-yl | Smp. 220-222 |
| 9 | $CH_3$ | $CH_3$ | H | H | S | 4 | 3-Methylpyrazin-2-yl | Smp. 167-169 |
| 10 | $CH_3$ | $CH_3$ | H | H | O | 4 | 6-Chlorpyridazin-3-yl | Smp. 265-267 |
| 11 | $CH_3$ | $CH_3$ | H | H | O | 4 | 6-Methoxypyridazin-3-yl | Smp. 237 |
| 12 | $CH_3$ | $CH_3$ | H | H | O | 4 | 2-Isopropyl-6-methyl-pyrimidin-4-yl | Smp. 147-149 |
| 13 | $CH_3$ | $CH_3$ | H | H | O | 4 | 2-Methylthio-6-methyl-pyrimidin-4-yl | Smp. 204-205 |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 14 | $CH_3$ | $CH_3$ | H | H | O | 4 | 2-tert.Butyl-6-chlor-pyrimidin-4-yl | Smp. 158-160 |
| 15 | $CH_3$ | $CH_3$ | H | H | O | 4 | 3,5-Dichlorpyridazin-4-yl | |
| 16 | $CH_3$ | $CH_3$ | H | H | O | 4 | 3-Chlor-4-methylthio-pyridazin-5-yl | |
| 17 | $CH_3$ | $CH_3$ | H | H | O | 4 | 3,6-Dichlorpyridazin-4-yl | |
| 18 | $CH_3$ | $CH_3$ | H | H | O | 4 | 3-Chlor-4-methylthio-pyridazin-6-yl | |
| 19 | $CH_3$ | $CH_3$ | H | H | O | 4 | 3,5,6-Trichlor-pyridazin-4-yl | |
| 20 | $CH_3$ | $CH_3$ | H | H | O | 4 | 3-Chlor-4-methyl-pyridazin-6-yl | |
| 21 | $OCH_3$ | $CH_3$ | H | H | O | 4 | 3-Methylpyrazin-2-yl | |
| 22 | $CH_3$ | $CH_3$ | H | H | S | 4 | 6-Chlorpyridazin-3-yl | |
| 23 | $OCH_3$ | $CH_3$ | H | H | O | 4 | 6-Methoxypyridazin-3-yl | |
| 24 | $CH_3$ | $CH_3$ | 3-$OCH_3$ | H | O | 4 | 6-Methoxypyridazin-3-yl | |
| 25 | $CH_3$ | $CH_3$ | H | H | O | 4 | 6-Methylthiopyridazin-3-yl | |
| 26 | $CH_3$ | $CH_3$ | H | H | O | 4 | 6-Methylaminopyridazin-3-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 27 | $CH_3$ | $CH_3$ | H | H | O | 4 | 2-Methyl-6-chlor-pyrimidin-4-yl | Smp. 222-223 |
| 28 | $CH_3$ | $CH_3$ | H | H | O | 4 | 2-Methylthio-6-chlor-pyrimidin-4-yl | |
| 29 | $CH_3$ | $CH_3$ | H | H | O | 4 | 2-Methylthio-5-phenyl-6-chlor-pyrimidin-4-yl | Smp.207-208 |
| 30 | $CH_3$ | $CH_3$ | H | H | O | 4 | 2-Methylthiopyrimidin-4-yl | Smp.191-193 |
| 31 | $OCH_3$ | $CH_3$ | H | H | O | 4 | 2-Methylthio-6-methyl-pyrimidin-4-yl | |
| 32 | $CH_3$ | $CH_3$ | H | H | S | 4 | 2-tert.-Butyl-6-chlor-pyrimidin-4-yl | |
| 33 | $CH_3$ | $CH_3$ | H | H | O | 4 | 4-Chlor-6-methyl-pyrimidin-2-yl | |
| 34 | $CH_3$ | $CH_3$ | H | H | O | 4 | 4-Methoxy-6-methyl-pyrimidin-2-yl | |
| 35 | $CH_3$ | $CH_3$ | H | H | O | 4 | 4,6-Dichlorpyrimidin-2-yl | |
| 36 | $CH_3$ | $CH_3$ | H | H | O | 4 | 2,6-Dimethylpyrimidin-4-yl | Smp.226-227 |
| 37 | $CH_3$ | $CH_3$ | H | H | O | 4 | 4-Chlor-5,6-dimethyl-pyrimidin-2-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 38 | $CH_3$ | $CH_3$ | H | H | O | 4 | 4-Methylpyrimidin-2-yl | Smp.196-198 |
| 39 | $CH_3$ | $CH_3$ | H | H | O | 4 | 4-Chlor-6-dimethyl-amino-1,3,5-triazin-2-yl | Smp. 230 |
| 40 | $CH_3$ | $CH_3$ | H | H | O | 4 | 4-Chlor-5-trifluor-methyl-pyrimidin-2-yl | |
| 41 | $CH_3$ | $CH_3$ | H | H | O | 4 | 2-Chlor-5-trifluor-methyl-pyrimidin-4-yl | |
| 42 | $CH_3$ | $CH_3$ | H | H | O | 4 | 6-Trifluormethyl-pyrimidin-4-yl | Smp.178-179 |
| 43 | $CH_3$ | $CH_3$ | H | H | O | 4 | 4-Chlor-6-trifluor-methyl-pyrimidin-2-yl | |
| 44 | $CH_3$ | $CH_3$ | H | H | O | 4 | 4-Methyl-6-trifluor-methyl-pyrimidin-2-yl | Smp.168-170 |
| 45 | $CH_3$ | $CH_3$ | H | H | O | 4 | 4-Methylamino-6-tri-fluormethyl-pyrimidin-2-yl | |
| 46 | $CH_3$ | $CH_3$ | H | H | O | 4 | 4-Trifluormethyl-pyrimidin-2-yl | Smp.191-192 |
| 47 | $CH_3$ | $CH_3$ | H | H | O | 4 | 2-Trifluormethyl-6-chlor-pyrimidin-4-yl | Smp.173-175 |
| 48 | $CH_3$ | $CH_3$ | H | H | O | 4 | 2-Trifluormethyl-pyrimidin-4-yl | Smp.224-225 |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 49 | $CH_3$ | $CH_3$ | H | H | O | 4 | 2-Trifluormethyl-6-methyl-pyrimidin-4-yl | Smp.209-210 |
| 50 | $CH_3$ | $CH_3$ | H | H | S | 4 | 4,6-Dimethylpyrimidin-2-yl | |
| 51 | $CH_3$ | $CH_3$ | H | H | S | 4 | 4-Chlorpyrimidin-2-yl | |
| 52 | $CH_3$ | $CH_3$ | H | H | S | 4 | 6-Chlorpyrimidin-4-yl | |
| 53 | $CH_3$ | $CH_3$ | H | H | S | 4 | 2-Isopropyl-6-methyl-pyrimidin-4-yl | |
| 54 | $CH_3$ | $CH_3$ | H | H | S | 4 | 2-Methylthio-6-methyl-pyrimidin-4-yl | |
| 55 | $CH_3$ | $CH_3$ | H | H | S | 4 | 4-Chlor-6-trifluor-methyl-pyrimidin-2-yl | |
| 56 | $CH_3$ | $CH_3$ | H | H | S | 4 | 4-Methyl-6-trifluor-methyl-pyrimidin-2-yl | Smp.195-197 |
| 57 | $CH_3$ | $CH_3$ | H | H | S | 4 | 2-Trifluormethyl-pyrimidin-4-yl | Smp.173-174 |
| 58 | $CH_3$ | $CH_3$ | H | H | S | 4 | 2-Trifluormethyl-6-methoxy-pyrimidin-4-yl | |
| 59 | $CH_3$ | $CH_3$ | $4-OCH_3$ | H | S | 3 | 6-Trifluormethyl-pyrimidin-4-yl | Smp.195-196 |
| 60 | $CH_3$ | $CH_3$ | $4-OCH_3$ | H | S | 3 | 6-Chlorpyrimidin-4-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 61 | $CH_3$ | $CH_3$ | $3-OCH_3$ | H | S | 4 | 4-Trifluormethyl-pyrimidin-2-yl | Smp.180-184 |
| 62 | $CH_3$ | $CH_3$ | $2-CH_3$ | H | S | 4 | 2,6-Dimethylpyrimidin-4-yl | |
| 63 | $CH_3$ | $CH_3$ | $2-CH_3$ | $6-CH_3$ | S | 4 | 4-Chlorpyrimidin-2-yl | |
| 64 | $OCH_3$ | $CH_3$ | H | H | O | 4 | 6-Chlorpyrimidin-4-yl | |
| 65 | $OCH_3$ | $CH_3$ | H | H | O | 4 | 2-Methylthio-pyrimidin-4-yl | |
| 66 | $OCH_3$ | $CH_3$ | H | H | O | 4 | 6-Trifluormethyl-pyrimidin-4-yl | |
| 67 | $CH_3$ | $CH_3$ | H | H | O | 4 | 5-Trifluormethyl-pyrimidin-2-yl | |
| 68 | $CH_2-CH=CH_2$ | $CH_3$ | H | H | O | 4 | 6-Chlorpyrimidin-4-yl | |
| 69 | Cyclopropyl | $CH_3$ | H | H | O | 4 | 6-Methylpyrimidin-4-yl | |
| 70 | $C_2H_5$ | $CH_3$ | H | H | O | 4 | 4-Trifluormethyl-pyrimidin-2-yl | |
| 71 | $CH_3$ | $CH_3$ | $2-CH_3$ | H | O | 4 | 4-Chlorpyrimidin-2-yl | |
| 72 | $CH_3$ | $CH_3$ | $2-isoC_3H_7$ | H | O | 4 | 6-Trifluormethyl-pyrimidin-4-yl | Smp.149-151 |
| 73 | $CH_3$ | $CH_3$ | $2-CH_3$ | $6-CH_3$ | O | 4 | 2-Trifluormethyl-pyrimidin-4-yl | Smp.196-197 |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 74 | $CH_3$ | $CH_3$ | $4-OCH_3$ | H | O | 3 | 2-Methyl-6-chlor-pyrimidin-4-yl | |
| 75 | $CH_3$ | $CH_3$ | $4-OCH_3$ | H | O | 3 | 6-Trifluormethyl-pyrimidin-4-yl | Smp.196-197 |
| 76 | $CH_3$ | $CH_3$ | $3-OCH_3$ | H | O | 4 | 2-Trifluormethyl-pyrimidin-4-yl | Smp.218-220 |
| 77 | $CH_3$ | $CH_3$ | $3-OCH_3$ | H | O | 4 | 2-Methyl-6-chlor-pyrimidin-4-yl | Smp. 204-208 |
| 78 | $CH_3$ | $CH_3$ | H | H | O | 4 | 2-Methylthio-6-trifluormethyl-pyrimidin-4-yl | Smp.161-163 |
| 79 | $CH_3$ | $CH_3$ | H | H | O | 4 | 2-Methyl-6-trifluormethyl-pyrimidin-4-yl | Smp.171-173 |
| 80 | $CH_3$ | $CH_3$ | $2-isoC_3H_7$ | H | S | 4 | 6-Trifluormethyl-pyrimidin-4-yl | Smp.157-158 |
| 81 | $OCH_3$ | $CH_3$ | $4-OCH_3$ | H | O | 3 | 6-Trifluormethyl-pyrimidin-4-yl | |
| 82 | $CH_2-CH=CH_2$ | $CH_3$ | H | H | O | 4 | 4-Trifluormethyl-pyrimidin-2-yl | |
| 83 | $OCH_3$ | $CH_3$ | H | H | O | 4 | 4-Trifluormethyl-pyrimidin-2-yl | |
| 84 | $CH_3$ | $CH_3$ | $2-CH_3$ | $6-CH_3$ | S | 4 | 2-Trifluormethyl-pyrimidin-4-yl | Smp.179-181 |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 85 | $CH_3$ | $CH_3$ | H | H | O | 4 | 2-Trifluormethyl-6-methoxy-pyrimidin-4-yl | |
| 86 | $CH_3$ | $CH_3$ | H | H | O | 4 | 2-Trifluormethyl-6-methylthio-pyrimidin-4-yl | |
| 87 | $CH_3$ | $CH_3$ | H | H | O | 4 | 2-Trifluormethyl-6-methylamino-pyrimidin-4-yl | |
| 88 | $CH_3$ | $CH_3$ | H | H | O | 4 | 2-Trifluormethyl-6-dimethylamino-pyrimidin-4-yl | |
| 89 | $CH_3$ | $CH_3$ | H | H | S | 4 | 2-Trifluormethyl-6-chlor-pyrimidin-4-yl | |
| 90 | Cyclopropyl | $CH_3$ | H | H | O | 4 | 4-Trifluormethyl-pyrimidin-2-yl | |
| 91 | $CH_3$ | $CH_3$ | H | H | S | 4 | 4-Methoxy-6-methyl-1,3,5-triazin-2-yl | |
| 92 | $CH_3$ | $CH_3$ | H | H | O | 4 | 4-Chlor-6-methyl-1,3,5-triazin-2-yl | |
| 93 | $CH_3$ | $CH_3$ | H | H | O | 4 | 4-Ethoxy-6-methyl-1,3,5-triazin-2-yl | |
| 94 | $CH_3$ | $CH_3$ | H | H | O | 4 | 4,6-Dichlor-1,3,5-triazin-2-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 95 | $CH_3$ | $CH_3$ | H | H | O | 4 | 4-Ethoxy-6-methoxy-1,3,5-triazin-2-yl | |
| 96 | $CH_3$ | $CH_3$ | H | H | O | 4 | 4-Methyl-1,3,5-triazin-2-yl | |
| 97 | $CH_3$ | $CH_3$ | H | H | O | 4 | 4-Chlor-6-trifluormethyl-1,3,5-triazin-2-yl | |
| 98 | $CH_3$ | $CH_3$ | H | H | O | 4 | 4-Chlormethyl-6-methyl-1,3,5-triazin-2-yl | |
| 99 | $CH_3$ | $CH_3$ | H | H | O | 4 | 4-Methyl-6-tert.-butyl-1,3,5-triazin-2-yl | |
| 100 | $CH_3$ | $CH_3$ | H | H | O | 4 | 4-Chlormethyl-6-methoxy-1,3,5-triazin-2-yl | |
| 101 | $CH_3$ | $CH_3$ | H | H | O | 4 | 4-Methylthio-1,3,5-triazin-2-yl | |
| 102 | $CH_3$ | $CH_3$ | H | H | O | 4 | 4-Methoxy-6-methyl-1,3,5-triazin-2-yl | |
| 103 | $OCH_3$ | $CH_3$ | H | H | O | 4 | 4-Ethyl-6-methoxy-1,3,5-triazin-2-yl | |
| 104 | $CH_3$ | $CH_3$ | H | H | O | 4 | 4-Methyl-6-trifluormethyl-1,3,5-triazin-2-yl | |

- 36 -

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 105 | $CH_3$ | $CH_3$ | H | H | O | 4 | 4-Methoxy-5-methyl-1,2,4-triazin-3-yl | |
| 106 | $CH_3$ | $CH_3$ | H | H | O | 4 | 4-Chlor-6-methoxy-1,3,5-triazin-2-yl | |
| 107 | $CH_3$ | $CH_3$ | H | H | O | 4 | 4,6-Dimethoxy-1,3,5-triazin-2-yl | |
| 108 | $CH_3$ | $CH_3$ | H | H | O | 4 | 4-Methoxy-1,3,5-triazin-2-yl | |
| 109 | $CH_3$ | $CH_3$ | H | H | O | 3 | 4-Methoxy-6-methyl-1,3,5-triazin-2-yl | |
| 110 | $CH_3$ | $CH_3$ | H | H | O | 4 | 4-Chlor-1,3,5-triazin-2-yl | |
| 111 | $CH_3$ | $CH_3$ | H | H | O | 4 | 4-Methoxy-6-methylthio-1,3,5-triazin-2-yl | |
| 112 | $CH_3$ | $CH_3$ | H | H | S | 4 | 3,5-Dichlor-1,2,4-triazin-6-yl | |
| 113 | $CH_3$ | $CH_3$ | H | H | O | 4 | 4-Cyclopropyl-6-methoxy-1,3,5-triazin-2-yl | |
| 114 | $CH_3$ | $CH_3$ | H | H | O | 4 | 4-Methyl-6-methylthio-1,3,5-triazin-2-yl | |
| 115 | $CH_3$ | $CH_3$ | H | H | O | 4 | 4-Ethyl-6-methyl-1,3,5-triazin-2-yl | |

Tabelle 1 (Fortsetzung

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 116 | $CH_3$ | $CH_3$ | H | H | O | 4 | 4-Dimethylamino-6-(2,2,2-trifluorethoxy)-1,3,5-triazin-2-yl | |
| 117 | $CH_3$ | $CH_3$ | H | H | O | 4 | 4-Isopropyl-6-methyl-1,3,5-triazin-2-yl | |
| 118 | $CH_3$ | $CH_3$ | H | H | O | 4 | 4-Methoxy-6-trifluormethyl-1,3,5-triazin-2-yl | |
| 119 | $OCH_3$ | $CH_3$ | H | H | O | 4 | 4-Methoxy-6-methyl-1,3,5-triazin-2-yl | |
| 120 | $CH_3$ | $CH_3$ | H | H | O | 4 | 4-Allyl-6-methyl-1,3,5-triazin-2-yl | |
| 121 | $CH_3$ | $CH_3$ | H | H | O | 4 | 4-Methylamino-6-trifluormethyl-1,3,5-triazin-2-yl | |
| 122 | $CH_3$ | $CH_3$ | 3-$OCH_3$ | H | O | 4 | 4,6-Dimethyl-1,3,5-triazin-2-yl | |
| 123 | $CH_3$ | $CH_3$ | H | H | O | 4 | 4-Trifluormethyl-1,3,5-triazin-2-yl | |
| 124 | $CH_3$ | $CH_3$ | H | H | O | 4 | 4,6-Dimethyl-1,3,5-triazin-2-yl | |

<u>Tabelle 1</u> (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 125 | $CH_3$ | $CH_3$ | H | H | O | 4 | 4-Methylamino-6-methoxy-1,3,5-triazin-2-yl | |
| 126 | $CH_3$ | $CH_3$ | H | H | O | 4 | 3,6-Dimethyl-1,2,4-triazin-5-yl | |
| 127 | $CH_3$ | $CH_3$ | H | H | O | 4 | 4-(Difluormethylthio)-6-methyl-1,3,5-triazin-2-yl | |
| 128 | $CH_3$ | $CH_3$ | H | H | O | 4 | 4-Methylamino-6-tert.-butyl-1,3,5-triazin-2-yl | |
| 129 | $CH_3$ | $CH_3$ | H | H | O | 4 | 4-Isopropyloxy-6-methoxy-1,3,5-triazin-2-yl | |
| 130 | $CH_3$ | $CH_3$ | H | H | O | 4 | 4-Cyclopropyl-6-methylamino-1,3,5-triazin-2-yl | |
| 131 | $CH_3$ | $CH_3$ | H | H | O | 4 | 4-Chlor-6-methylthio-1,3,5-triazin-2-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 132 | $CH_3$ | $CH_3$ | H | H | O | 4 | 3,5-Dichlor-1,2,4-triazin-6-yl | |
| 133 | $CH_3$ | $CH_3$ | H | H | O | 4 | 4-Allyl-1,3,5-triazin-2-yl | |
| 134 | $CH_3$ | $CH_3$ | H | H | O | 4 | 4-Methoxy-6-(2,2,2-trifluorethoxy)-1,3,5-triazin-2-yl | |
| 135 | $CH_3$ | $CH_3$ | H | H | O | 4 | 4-Fluormethyl-6-methoxy-1,3,5-triazin-2-yl | |
| 136 | $CH_3$ | $CH_3$ | H | H | S | 4 | 2,6-Dimethylpyrimidin-4-yl | Smp.205-206 |
| 137 | $CH_3$ | $CH_3$ | H | H | O | 4 | 4-Chlor-6-ethylamino-1,3,5-triazin-2-yl | |
| 138 | $CH_3$ | $CH_3$ | H | H | O | 4 | 4,6- Bis(methylthio)-1,3,5-triazin-2-yl | |
| 139 | $CH_3$ | $CH_3$ | H | H | O | 4 | 5-Chlorpyrimidin-2-yl | Smp.212-213 |
| 140 | $CH_3$ | $CH_3$ | $3-OCH_3$ | H | O | 4 | 5-Methyl-4-trifluormethyl-pyrimidin-2-yl | Smp.200-201 |
| 141 | $CH_3$ | $CH_3$ | $2-CH_3$ | $6-CH_3$ | O | 4 | Pyrazin-2-yl | Smp. >240 |
| 142 | $CH_3$ | $CH_3$ | H | H | O | 3 | 6-Chlorpyridazin-3-yl | |

-39-

<u>Tabelle 1</u> (Fortsetzung)

| Verb. Nr. | $R_1$ | $R_2$ | $R_4$ | $R_5$ | X | Position $-O-R_3$ | $R_3$ | Physikal. Konst.[°C] |
|---|---|---|---|---|---|---|---|---|
| 143 | $CH_3$ | $CH_3$ | H | H | O | 4 | 2-Phenyl-6-chlor-pyrimidin-4-yl | |
| 144 | $CH_3$ | $CH_3$ | H | H | O | 4 | 5-Phenyl-4-trifluor-methyl-pyrimidin-2-yl | Smp.180-181 |
| 145 | $CH_3$ | $CH_3$ | $4-OCH_3$ | H | O | 3 | 5-Methyl-4-trifluorme-thyl-pyrimidin-2-yl | Smp.170-172 |
| 146 | $CH_3$ | $CH_3$ | $3-C_2H_5$ | H | O | 4 | 6-Methyl-4-trifluorme-thyl-pyrimidin-2-yl | |
| 147 | $CH_3$ | $CH_3$ | $2-n-C_3H_7$ | H | S | 4 | 6-Methyl-4-trifluorme-thyl-pyrimidin-2-yl | |
| 148 | $OCH_3$ | $CH_3$ | H | H | O | 4 | 5-Chlorpyrimidin-2-yl | Smp.192-194 |
| 149 | $CH_3$ | $CH_3$ | H | H | S | 4 | 2-Methylthio-6-triflu-ormethyl-pyrimidin-4-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 150 | $CH_3$ | $CH_3$ | H | H | O | 4 | 2,6-Bis-(trifluormethyl)-pyrimidin-4-yl | Smp. 159-160 |
| 151 | $CH_3$ | $CH_3$ | 2-n-$C_3H_7$ | H | O | 4 | 6-Chlor-2-trifluormethyl-pyrimidin-4-yl | |
| 152 | $CH_3$ | $CH_3$ | H | H | S | 4 | 6-Methyl-2-trifluormethyl-pyrimidin-4-yl | |
| 153 | $CH_3$ | $CH_3$ | H | H | O | 4 | 6-Chlor-1-oxy-pyridazin-3-yl | Smp. 267-269 |
| 154 | $CH_3$ | $CH_3$ | 4-$OC_2H_5$ | H | S | 3 | 6-Trifluormethyl-pyrimidin-4-yl | |
| 155 | $CH_2-CH=CH_2$ | $CH_3$ | H | H | O | 4 | 6-Trifluormethyl-pyrimidin-4-yl | |
| 156 | $C_2H_5$ | $CH_3$ | H | H | S | 4 | 6-Trifluormethyl-pyrimidin-4-yl | |
| 157 | $C_2H_5$ | $CH_3$ | H | H | O | 4 | 6-Methyl-2-trifluormethyl-pyrimidin-4-yl | |

Tabelle 1 (Fortsetzung)

| Verb. Nr. | $R_1$ | $R_2$ | $R_4$ | $R_5$ | X | Position $-O-R_3$ | $R_3$ | Physikal. Konst. [°C] |
|---|---|---|---|---|---|---|---|---|
| 158 | $CH_3$ | $CH_3$ | H | H | S | 4 | 5-Trifluormethyl-pyrimidin-2-yl | |
| 159 | $CH_2-CH=CH_2$ | $CH_3$ | H | H | S | 4 | 5-Trifluormethyl-pyrimidin-2-yl | |
| 160 | $C_2H_5$ | $CH_3$ | H | H | O | 4 | 5-Trifluormethyl-pyrimidin-2-yl | |
| 161 | $CH_3$ | $CH_3$ | $4-OC_2H_5$ | H | S | 3 | 5-Trifluormethyl-pyrimidin-2-yl | |
| 162 | $CH_3$ | $CH_3$ | H | H | S | 4 | 4-Chlor-6-trifluormethyl-1,3,5-triazin-2-yl | |
| 163 | $CH_3$ | $CH_3$ | H | H | S | 4 | 4-Trifluormethyl-1,3,5-triazin-2-yl | |

<u>Tabelle 1</u> (Fortsetzung)

| Verb. Nr. | $R_1$ | $R_2$ | $R_4$ | $R_5$ | X | Position $-O-R_3$ | $R_3$ |
|---|---|---|---|---|---|---|---|
| 164 | iso-$C_3H_7$ | $CH_3$ | H | H | O | 4 | 4-Trifluormethyl-pyrimidin-2-yl |
| 165 | iso-$C_3H_7$ | $CH_3$ | H | H | S | 4 | 4-Trifluormethyl-pyrimidin-2-yl |
| 166 | $CH_3$ | $CH_3$ | H | H | S | 4 | 4-Trifluormethyl-pyrimidin-2-yl |
| 167 | $CH_2-CH=CH_2$ | $CH_3$ | H | H | S | 4 | 4-Trifluormethyl-pyrimidin-2-yl |
| 168 | Cyclopropyl | $CH_3$ | H | H | S | 4 | 4-Trifluormethyl-pyrimidin-2-yl |
| 169 | $CH_3$ | $CH_3$ | 2-$OCH_3$ | H | O | 4 | 4-Trifluormethyl-pyrimidin-2-yl |

Tabelle 1 (Fortsetzung)

| Verb. Nr. | $R_1$ | $R_2$ | $R_4$ | $R_5$ | X | Position $-O-R_3$ | $R_3$ |
|---|---|---|---|---|---|---|---|
| 170 | $CH_3$ | $CH_3$ | $3-OC_2H_5$ | H | S | 4 | 6-Trifluormethyl-pyrimidin-4-yl |
| 171 | $CH_3$ | $CH_3$ | $4-OC_2H_5$ | H | S | 3 | 6-Trifluormethyl-pyrimidin-4-yl |
| 172 | $CH_3$ | $CH_3$ | $4-CH_3$ | H | O | 3 | 6-Trifluormethyl-pyrimidin-4-yl |
| 173 | $CH_3$ | $CH_3$ | $4-CH_3$ | H | O | 2 | 6-Trifluormethyl-pyrimidin-4-yl |
| 174 | $CH_3$ | $CH_3$ | $4-CH_3$ | H | S | 2 | 6-Trifluormethyl-pyrimidin-4-yl |
| 175 | $CH_2-CH=CH_2$ | $CH_3$ | $4-CH_3$ | H | O | 2 | 6-Trifluormethyl-pyrimidin-4-yl |
| 176 | iso-$C_3H_7$ | $CH_3$ | $4-CH_3$ | H | O | 2 | 6-Trifluormethyl-pyrimidin-4-yl |
| 177 | $CH_3$ | $CH_3$ | $2-OCH_3$ | H | O | 4 | 6-Trifluormethyl-pyrimidin-4-yl |

<u>Tabelle 1</u> (Fortsetzung)

| Verb. Nr. | $R_1$ | $R_2$ | $R_4$ | $R_5$ | X | Position $-O-R_3$ | $R_3$ |
|---|---|---|---|---|---|---|---|
| 178 | $CH_2-CH=CH_2$ | $CH_3$ | H | H | O | 4 | 2-Methyl-6-trifluormethyl-pyrimidin-4-yl |
| 179 | $C_2H_5$ | $CH_3$ | H | H | O | 4 | 2-Methyl-6-trifluormethyl-pyrimidin-4-yl |
| 180 | $CH_3$ | $CH_3$ | H | H | S | 4 | 2-Methyl-6-trifluormethyl-pyrimidin-4-yl |
| 181 | $CH_3$ | $CH_3$ | $4-CH_3$ | H | O | 3 | 2-Methyl-6-trifluormethyl-pyrimidin-4-yl |
| 182 | $CH_3$ | $CH_3$ | $4-CH_3$ | H | S | 3 | 2-Methyl-6-trifluormethyl-pyrimidin-4-yl |
| 183 | $CH_2-CH=CH_2$ | $CH_3$ | $4-CH_3$ | H | O | 3 | 2-Methyl-6-trifluormethyl-pyrimidin-4-yl |
| 184 | iso-$C_3H_7$ | $CH_3$ | $4-CH_3$ | H | S | 3 | 2-Methyl-6-trifluormethyl-pyrimidin-4-yl |

Tabelle 1 (Fortsetzung)

| Verb. Nr. | $R_1$ | $R_2$ | $R_4$ | $R_5$ | X | Position $-O-R_3$ | $R_3$ |
|---|---|---|---|---|---|---|---|
| 185 | $CH_3$ | $CH_3$ | H | H | O | 4 | 2-Isopropyl-6-trifluormethyl-pyrimidin-4-yl |
| 186 | $CH_3$ | $CH_3$ | $2-CH_3$ | $6-CH_3$ | S | 4 | 2-Isopropyl-6-trifluromethyl-pyrimidin-4-yl |
| 187 | $CH_3$ | $CH_3$ | H | H | O | 4 | 2-tert-Butyl-6-trifluormethyl-pyrimidin-4-yl |
| 188 | $CH_3$ | $CH_3$ | H | H | S | 4 | 2-tert-Butyl-6-trifluormethyl-pyrimidin-4-yl |
| 189 | $CH_3$ | $CH_3$ | H | H | O | 4 | 2-Cyclopropyl-6-trifluormethyl-pyrimidin-4-yl |
| 190 | $CH_3$ | $CH_3$ | H | H | S | 4 | 2-Cyclopropyl-6-trifluormethyl-pyrimidin-4-yl |

Tabelle 2: Verbindungen der Formel III

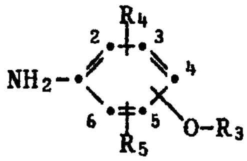

| Verb. Nr. | $R_4$ | $R_5$ | Position $-O-R_3$ | $R_3$ | Physikal. Konst.[°C] |
|---|---|---|---|---|---|
| 2.1 | H | H | 4 | 3-Methylpyrazin-2-yl | Smp.108-109 |
| 2.2 | H | H | 4 | 3-Chlorpyrazin-2-yl | Smp.130-131 |
| 2.3 | H | H | 4 | 6-Chlorchinoxalin-2-yl | Smp.107-112 |
| 2.4 | H | H | 4 | Pyrimidin-2-yl | Smp.125-129 |
| 2.5 | H | H | 4 | 4,6-Dimethyl-pyrimidin-2-yl | Smp.92-96 |
| 2.6 | H | H | 4 | 4-Chlorpyrimidin-2-yl | Oel |
| 2.7 | H | H | 4 | 6-Chlorpyrimidin-4-yl | Oel |
| 2.8 | H | H | 4 | Chinolin-2-yl | |
| 2.9 | H | H | 4 | 6-Chlorpyridazin-3-yl | |
| 2.10 | H | H | 4 | 6-Methoxypyridazin-3-yl | |
| 2.11 | H | H | 4 | 2-Isopropyl-6-methyl-pyrimidin-4-yl | |
| 2.12 | H | H | 4 | 2-Methylthio-6-methylpyrimidin-4-yl | Oel |
| 2.13 | H | H | 4 | 2-tert.Butyl-6-chlor-pyrimidin-4-yl | Oel |
| 2.14 | H | H | 4 | 2-Methyl-6-chlor-pyrimidin-4-yl | |
| 2.15 | H | H | 4 | 2-Methylthio-5-phenyl-6-chlor-pyrimidin-4-yl | |
| 2.16 | H | H | 4 | 2-Methylthio-pyrimidin-4-yl | |
| 2.17 | H | H | 4 | 2,6-Dimethyl-pyrimidin-4-yl | |
| 2.18 | H | H | 4 | 4-Methylpyrimidin-2-yl | |

Tabelle 2 (Fortsetzung)

| Verb. Nr. | $R_4$ | $R_5$ | Position $-O-R_3$ | $R_3$ | Physikal. Konst. [°C] |
|---|---|---|---|---|---|
| 2.19 | H | H | 4 | 4-Chlor-6-dimethyl-amino-1,3,5-triazin-2-yl | |
| 2.20 | H | H | 4 | 6-Trifluormethyl-pyrimidin-4-yl | |
| 2.21 | H | H | 4 | 4-Methyl-6-trifluor-methyl-pyrimidin-2-yl | |
| 2.22 | H | H | 4 | 4-Trifluormethyl-pyrimidin-2-yl | |
| 2.23 | H | H | 4 | 2-Trifluormethyl-6-chlor-pyrimidin-4-yl | |
| 2.24 | H | H | 4 | 2-Trifluormethyl-pyrimidin-4-yl | |
| 2.25 | H | H | 4 | 2-Trifluormethyl-6-methyl-pyrimidin-4-yl | |
| 2.26 | $4-OCH_3$ | H | 3 | 6-Trifluormethyl-pyrimidin-4-yl | |
| 2.27 | $3-OCH_3$ | H | 4 | 4-Trifluormethyl-pyrimidin-2-yl | |
| 2.28 | $2-isoC_3H_7$ | H | 4 | 6-Trifluormethyl-pyrimidin-4-yl | |
| 2.29 | $2-CH_3$ | $6-CH_3$ | 4 | 2-Trifluormethyl-pyrimidin-4-yl | |
| 2.30 | $3-OCH_3$ | H | 4 | 2-Trifluormethyl-pyrimidin-4-yl | |
| 2.31 | $3-OCH_3$ | H | 4 | 2-Methyl-6-chlor-pyrimidin-4-yl | |
| 2.32 | H | H | 4 | 2-Methylthio-6-tri-fluormethyl-pyrimidin-4-yl | |
| 2.33 | H | H | 4 | 2-Methyl-6-trifluor-methyl-pyrimidin-4-yl | |

Tabelle 2 (Fortsetzung)

| Verb. Nr. | $R_4$ | $R_5$ | Position $-O-R_3$ | $R_3$ | Physikal. Konst. [°C] |
|---|---|---|---|---|---|
| 2.34 | H | H | 4 | 5-Chlorpyrimidin-2-yl | |
| 2.35 | $3-OCH_3$ | H | 4 | 5-Methyl-4-trifluor-methylpyrimidin-2-yl | |
| 2.36 | $2-CH_3$ | $6-CH_3$ | 4 | Pyrazin-2-yl | |
| 2.37 | H | H | H | 5-Phenyl-4-trifluor-methyl-pyrimidin-2-yl | |
| 2.38 | $4-OCH_3$ | H | 3 | 5-Methyl-4-trifluor-methyl-pyrimidin-2-yl | |
| 2.39 | H | H | 4 | 2,6-Bis-(trifluor-methyl)-pyrimidin-4-yl | |
| 2.40 | H | H | 4 | 6-Chlor-1-oxy-pyridazin-3-yl | |

3. Formulierungsbeispiele (% = Gewichtsprozent)

| 3.1. Emulsions-Konzentrate | a) | b) | c) |
|---|---|---|---|
| Wirkstoff aus Tabelle I | 25 % | 40 % | 50 % |
| Ca-Dodecylbenzolsulfonat | 5 % | 8 % | 6 % |
| Ricinusöl-polyethylenglykolether (36 Mol Ethylenoxid) | 5 % | - | - |
| Tributylphenol-polyethylenglykolether (30 Mol Ethylenoxid) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| 3.2. Lösungen | a) | b) | c) | d) |
|---|---|---|---|---|
| Wirkstoff aus Tabelle I | 80 % | 10 % | 5 % | 95 % |
| Ethylenglykol-monomethyl-ether | 20 % | - | - | - |
| Polyethylenglykol MG 400 | - | 70 % | - | - |
| N-Methyl-2-pyrrolidon | - | 20 % | - | - |

| | | | | |
|---|---|---|---|---|
| Epoxydiertes Kokosnussöl | - | - | 1 % | 5 % |
| Benzin (Siedegrenze 160-190°C) | - | - | 94 % | - |

(MG = Molekulargewicht)

Die Lösungen sind zur Anwendung in Form kleinster Tropfen geeignet.

| 3.3. Granulate | a) | b) |
|---|---|---|
| Wirkstoff der Tabelle I | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum eingedampft. Solche Granulate können dem Viehfutter beigemischt werden.

| 3.4. Stäubemittel | a) | b) |
|---|---|---|
| Wirkstoff der Tabelle I | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

| 3.5. In Wasser dispergierbare Pulvermischung | a) | b) | c) |
|---|---|---|---|
| Wirkstoff aus Tabelle I | 25 % | 50 % | 75 % |
| Na-Ligninsulfonat | 5 % | 5 % | - |
| Oelsäure | 3 % | - | 5 % |
| Na-Diisobutylnaphthalinsulfonat | - | 6 % | 10 % |
| Octylphenolpolyethylenglykolether (7-8 Mol Ethylenoxid) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| 3.6. Emulsions-Konzentrat | a) | b) | c) |
|---|---|---|---|
| Wirkstoff aus Tabelle I | 10 % | 8 % | 60 % |
| Octylphenolpolyethylenglykolether (4-5 Mol Ethylenoxid) | 3 % | 3 % | 2 % |
| Ca-Dodecylbenzolsulfonat | 3 % | 4 % | 4 % |
| Ricinusölpolyglykolether (35 Mol Ethylenoxid) | 4 % | 5 % | 4 % |
| Cyclohexanon | 30 % | 40 % | 15 % |
| Xylolgemisch | 50 % | 40 % | 15 % |

Aus diesem Konzentrat können durch Verdünnen mit Wasser Emulsionen jeder gewünschten Konzentration hergestellt werden.

| 3.7. Stäubemittel | a) | b) |
|---|---|---|
| Wirkstoff aus Tabelle I | 5 % | 8 % |
| Talkum | 95 % | - |
| Kaolin | - | 92 % |

Man erhält anwendungsfertige Stäubemittel, indem der Wirkstoff mit dem Träger vermischt auf einer geeigneten Mühle vermahlen wird.

| 3.8. Granulat | |
|---|---|
| Wirkstoff aus Tabelle I | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

3.9. Granulat

| | |
|---|---|
| Wirkstoff aus Tabelle I | 3 % |
| Polyethylenglykol (MG 200) | 3 % |
| Kaolin | 94 % |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

3.10. Suspensions-Konzentrat

| | |
|---|---|
| Wirkstoff aus Tabelle I | 40 % |
| Ethylenglykol | 10 % |
| Nonylphenolpolyethylenglykolether (15 Mol Ethylenoxid) | 6 % |
| Na-Ligninsulfonat | 10 % |
| Carboxymethylcellulose | 1 % |
| 37%ige wässrige Formaldehyd-Lösung | 0,2 % |
| Silikonöl in Form einer 75%igen wässrigen Emulsion | 0,8 % |
| Wasser | 32 % |

Der fein gemahlene Wirkstoff wird mit den Zusatzstoffen innig vermischt. Man erhält so ein Suspension-Konzentrat, aus welchem durch Verdünnen mit Wasser Suspensionen jeder gewünschten Konzentration hergestellt werden können.

3.11. Pellets bzw. Boli

| | | |
|---|---|---|
| I | Wirkstoff der Tabelle I | 33,0 % |
| | Methylcellulose | 0,80 % |
| | Kieselsäure hochdispers | 0,80 % |
| | Maisstärke | 8,40 % |

| | | |
|---|---|---|
| II | Milchzucker krist. | 22,50 % |
| | Maisstärke | 17,00 % |
| | mikrokrist. Cellulose | 16,50 % |
| | Magnesiumstearat | 1,00 % |

I Methylcellulose in Wasser einrühren und quellen lassen; Kieselsäure in die Quellung einrühren und homogen suspendieren. Wirkstoff und Maisstärke mischen. In diese Mischung die wässrige Suspension einarbeiten und zu einem Teig kneten. Diese Masse durch ein Sieb 12 M granulieren und trocknen.

II Alle 4 Hilfsstoffe gut mischen.

III Phasen I und II mischen und zu Pellets oder Boli verpressen.

4. Biologisches Beispiel

Die anthelmintische Wirksamkeit wird anhand des folgenden Versuchs demonstriert:

4.1. Versuch an mit Nematoden wie Haemonchus contortus und Trichostrongylus colubriformis infizierten Schafen

Der Wirkstoff wird in Form einer Suspension mit einer Magensonde oder durch Pansen-Injektion Schafen verabreicht, die vorher mit Nematoden wie Haemonchus contortus und Trichostrongylus colubriformis künstlich infiziert wurden. Pro Versuch resp. pro Dosis werden 1 bis 3 Tiere verwendet. Jedes Schaf wird nur mit einer einzigen Dosis behandelt.

Eine erste Evaluierung erfolgt dadurch, dass die Anzahl der vor und nach der Behandlung im Kot der Schafe ausgeschiedenen Wurmeier verglichen wird.

Sieben bis zehn Tage nach der Behandlung werden die Schafe getötet und seziert. Die Auswertung erfolgt durch Auszählung der nach der Behandlung im Darm zurückbleibenden Würmer. Gleichzeitig und gleichartig infizierte aber unbehandelte Schafe dienen als Kontrolle resp. Vergleich.

Als Suspension eingesetzte Wirkstoffe aus Tabelle I bewirken bei Schafen in einer Dosis, welche bei 20 mg/kg Körpergewicht oder tiefer liegt, eine Reduzierung des Nematodenbefalls um 90 % oder mehr, verglichen mit unbehandelten, aber infizierten Vergleichsgruppen. So erhält man beispielsweise bei einer Dosis von 20 mg/kg Körpergewicht mit der Verbindung Nr. 1, 2, 9 und 76 eine Reduzierung des Nematodenbefalls um mindestens 90 %. Darüber hinaus bewirken die Verbindungen Nr. 42, 44, 46, 48, 49, 56, 57, 59, 61, 72, 73, 79, 80, 84, 139, 140, 144 und 153 bei einer Dosis von 10 mg/kg Körpergewicht eine Reduzierung des Nematodenbefalls von 90 % oder mehr.

Patentansprüche für die Vertragsstaaten: DE, FR, CH, LI, IT, NL, BE, SE, LU

1. 5-Phenylcarbamoylbarbitursäurederivate der allgemeinen Formel I

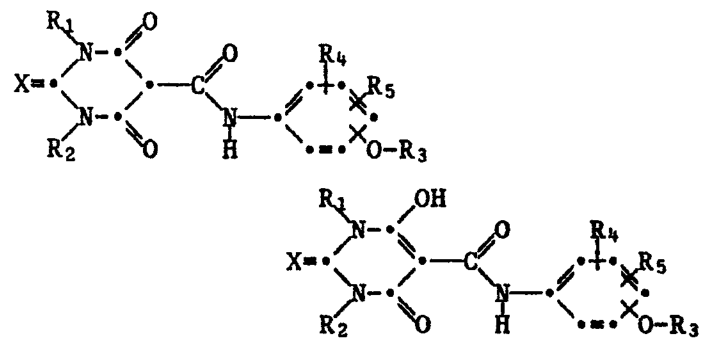

(I)

einschliesslich ihrer tautomeren Formen, Salze und N-oxyde, worin
$R_1$ $C_1$-$C_4$-Alkyl, $C_1$-$C_3$-Alkoxy, Allyl oder $C_3$-$C_6$-Cycloalkyl,
$R_2$ $C_1$-$C_4$-Alkyl oder Allyl,
$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und
X ein Sauerstoff- oder Schwefelatom bedeuten.

2. 5-Phenylcarbamoylbarbitursäurederivate der allgemeinen Formel I

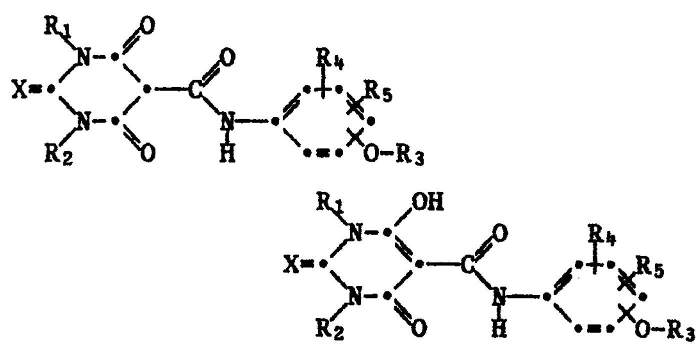

(I)

einschliesslich ihrer tautomeren Formen und Salze gemäss Anspruch 1, worin

$R_1$ $C_1$-$C_4$-Alkyl, $C_1$-$C_3$-Alkoxy, Allyl oder $C_3$-$C_6$-Cycloalkyl,

$R_2$ $C_1$-$C_4$-Alkyl oder Allyl,

$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und

X ein Sauerstoff- oder Schwefelatom bedeuten.

3. Verbindungen gemäss Anspruch 2, dadurch gekennzeichnet, dass $R_1$, $R_2$, $R_4$, $R_5$ und X die im Anspruch 2 angegebenen Bedeutungen haben und $R_3$ für einen cyclischen Rest, ausgewählt aus der Gruppe bestehend aus

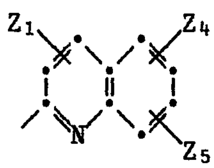 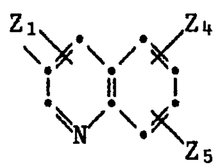 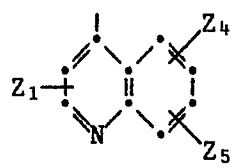 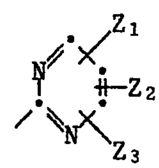

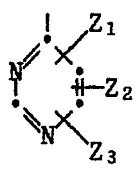 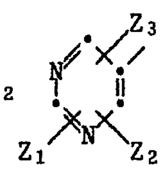 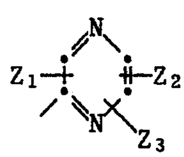 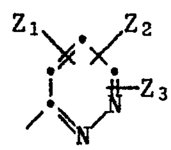 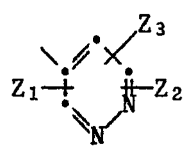

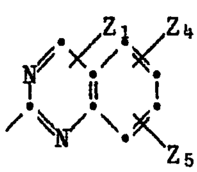 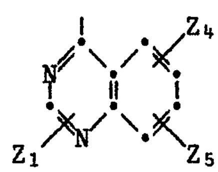 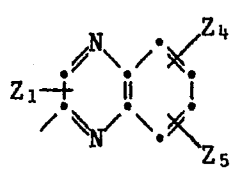 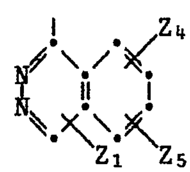

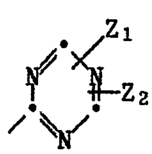 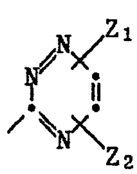 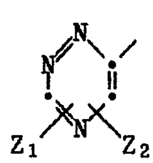 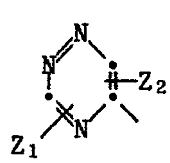 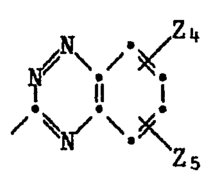

worin $Z_1$, $Z_2$, $Z_3$, $Z_4$ und $Z_5$ unabhängig voneinander Wasserstoff, $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cycloalkyl oder Phenyl bedeuten und $Z_1$, $Z_2$ und $Z_3$ am heteroaromatischen, $Z_4$ und $Z_5$ am ankondensierten homoaromatischen Ring stehen, steht.

4. Verbindungen gemäss Anspruch 1, dadurch gekennzeichnet, dass $R_1$ $C_1$-$C_4$-Alkyl, $C_1$-$C_3$-Alkoxy, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten.

5. Verbindungen gemäss Anspruch 4, dadurch gekennzeichnet, dass $R_1$ $C_1$-$C_4$-Alkyl, Allyl, Cyclopropyl oder $C_1$-$C_3$-Alkoxy, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten.

6. Verbindungen gemäss Anspruch 1, dadurch gekennzeichnet, dass $R_1$ $C_1$-$C_4$-Alkyl, Methoxy, Allyl oder Cyclopropyl, $R_2$ Methyl, $R_3$ einen unsubstituierten oder ein- bis dreifach durch Substituenten der Gruppe $C_1$-$C_4$-Alkyl, Methoxy, Methylamino, Dimethylamino, Methylthio, Trifluormethyl, Halogen oder Phenyl substituierten Pyrazinyl-,

Pyridazinyl-, Triazinyl-, Pyrimidinyl-, Chinoxalinyl-, Chinolyl- oder Chinazolinylrest, $R_4$ Wasserstoff, $C_1$-$C_4$-Alkyl oder Methoxy, $R_5$ Wasserstoff oder Methyl und X ein Sauerstoff- oder Schwefelatom bedeuten.

7. Verbindungen gemäss Anspruch 5, dadurch gekennzeichnet, dass $R_1$ $C_1$-$C_4$-Alkyl, Allyl, Cyclopropyl, oder Methoxy, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyrimidinyl-, Pyrazinyl- oder Pyridazinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten und der Molekülteil $-OR_3$ in meta- oder para-Stellung zum Stickstoffatom der Carbamoylgruppe steht.

8. Verbindungen gemäss Anspruch 5, dadurch gekennzeichnet, dass $R_1$ Methyl oder Methoxy, $R_2$ Methyl, $R_3$ einen unsubstituierten oder ein- oder zweifach durch Substituenten der Gruppe $C_1$-$C_4$-Alkyl, Halogen-$C_1$-$C_4$-Alkyl, Halogen oder Phenyl substituierten, über ein Kohlenstoffatom gebundenen Pyrimidinylring, $R_4$ Wasserstoff, $C_1$-$C_4$-Alkyl oder $C_1$-$C_4$-Alkoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht.

9. Verbindungen gemäss Anspruch 8, dadurch gekennzeichnet, dass $R_1$ Methyl, $R_2$ Methyl, $R_3$ einen ein- oder zweifach durch Substituenten der Gruppe Methyl, Trifluormethyl, Chlor oder Phenyl substituierten, über ein Kohlenstoffatom gebundenen Pyrimidinylring, $R_4$ Wasserstoff, Methyl, Isopropyl, Methoxy oder Aethoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht.

10. Verbindungen gemäss Anspruch 9, dadurch gekennzeichnet, dass $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyrimidinylring, $R_4$ Wasserstoff, Methyl, Isopropyl oder Methoxy, $R_5$ Wasserstoff oder Methyl und X ein Sauerstoff- oder Schwefelatom bedeuten und der Molekülteil $-OR_3$ in para-Stellung zum Stickstoffatom der Carbamoylgruppe steht.

11. Eine Verbindung gemäss Anspruch 1 aus der Gruppe:
1,3-Dimethyl-5-[4-(6-chlor-1-oxy-pyridazin-3-yl-oxy)-phenylcarbamoyl]-barbitursäure,
1,3-Dimethyl-5-[4-(3-methylpyrazin-2-yl-oxy)-phenylcarbamoyl]-barbitursäure,
1,3-Dimethyl-5-[4-(3-chlorpyrazin-2-yl-oxy)-phenylcarbamoyl]-barbitursäure,
1,3-Dimethyl-5-[4-(3-methylpyrazin-2-yl-oxy)-phenylcarbamoyl]-2-thiobarbitursäure,
1,3-Dimethyl-5-[4-(6-trifluormethylpyrimidin-4-yl-oxy)-phenylcarbamoyl]-barbitursäure,
1,3-Dimethyl-5-[4-(4-trifluormethylpyrimidin-2-yl-oxy)-phenylcarbamoyl]-barbitursäure,
1,3-Dimethyl-5-[4-(2-trifluormethylpyrimidin-4-yl-oxy)-phenylcarbamoyl]-2-thiobarbitursäure,
1,3-Dimethyl-5-[4-methoxy-3-(6-trifluormethylpyrimidin-4-yl-oxy)-phenylcarbamoyl]-2-thiobarbitursäure,
1,3-Dimethyl-5-[3-methoxy-4-(4-trifluormethylpyrimidin-2-yl-oxy)-phenylcarbamoyl]-2-thiobarbitursäure,
1,3-Dimethyl-5-[2-isopropyl-4-(6-trifluormethylpyrimidin-4-yl-oxy)-phenylcarbamoyl]-barbitursäure,
1,3-Dimethyl-5-[2,6-dimethyl-4-(2-trifluormethyl-pyrimidin-4-yl-oxy)-phenylcarbamoyl]-barbitursäure,
1,3-Dimethyl-5-[3-methoxy-4-(2-trifluormethylpyrimidin-4-yl-oxy)-phenylcarbamoyl]-barbitursäure,
1,3-Dimethyl-5-[4-(2-methyl-6-trifluormethylpyrimidin-4-yl-oxy)-phenylcarbamoyl]-barbitursäure,

1,3-Dimethyl-5-[2-isopropyl-4-(6-trifluormethylpyrimidin-4-yl-oxy)-phenylcarbamoyl]-2-thiobarbitursäure,

1,3-Dimethyl-5-[2,6-dimethyl-4-(2-trifluormethylpyrimidin-4-yl-oxy)-phenylcarbamoyl]-2-thiobarbitursäure,

1,3-Dimethyl-5-[4-(5-chlorpyrimidin-2-yl-oxy)-phenylcarbamoyl]-barbitursäure,

1,3-Dimethyl-5-[3-methoxy-4-(5-methyl-4-trifluormethylpyrimidin-2-yl-oxy)-phenylcarbamoyl]-barbitursäure,

1,3-Dimethyl-5-[4-(5-phenyl-4-trifluormethylpyrimidin-2-yl-oxy)-phenylcarbamoyl]-barbitursäure,

1,3-Dimethyl-5-[4-(4-methyl-6-trifluormethylpyrimidin-2-yl-oxy)-phenylcarbamoyl]-barbitursäure,

1,3-Dimethyl-5-[4-(4-methyl-6-trifluormethylpyrimidin-2-yl-oxy)-phenylcarbamoyl]-2-thiobarbitursäure,

1,3-Dimethyl-5-[4-(6-methyl-2-trifluormethylpyrimidin-4-yl-oxy)-phenylcarbamoyl]-barbitursäure und

1,3-Dimethyl-5-[4-(2-trifluormethylpyrimidin-4-yl-oxy)-phenylcarbamoyl]-barbitursäure.

12. Verfahren zur Herstellung von Verbindungen der Formel I gemäss Anspruch 1, dadurch gekennzeichnet, dass man

a) eine Verbindung der Formel II

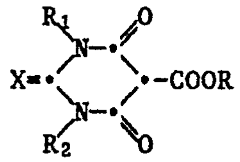

(II)

worin $R_1$, $R_2$ und X die für Formel I angegebenen Bedeutungen haben und R $C_1$-$C_5$-Alkyl oder unsubstituiertes oder durch Nitro substituiertes Phenyl bedeutet, mit einer Verbindung der Formel III

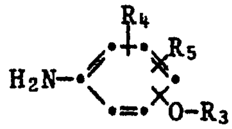

(III)

worin $R_3$, $R_4$ und $R_5$ die für Formel I angegebenen Bedeutungen haben, umsetzt, oder

b) eine Verbindung der Formel IV

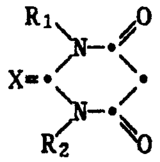

(IV)

worin $R_1$, $R_2$ und X die für Formel I angegebenen Bedeutungen haben, mit einer Verbindung der Formel V

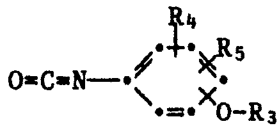

(V)

worin $R_3$, $R_4$ und $R_5$ die für Formel I angegebenen Bedeutungen haben, umsetzt, oder

c) eine Verbindung der Formel IV, worin $R_1$, $R_2$ und X die für Formel I angegebenen Bedeutungen haben, mit einer Verbindung der Formel VI

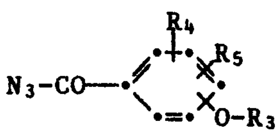

(VI)

worin $R_3$, $R_4$ und $R_5$ die für Formel I angegebenen Bedeutungen haben, umsetzt, oder

d) eine Verbindung der Formel VII

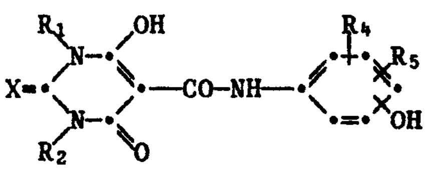

(VII)

worin $R_1$, $R_2$, $R_4$, $R_5$ und X die für Formel I angegebenen Bedeutungen haben, mit einer Verbindung der Formel VIII

$Q-R_3$ (VIII)

worin Q für eine übliche Abgangsgruppe steht und $R_3$ die für Formel I angegebene Bedeutung hat, in Gegenwart einer Base umsetzt, oder

e) zur Herstellung von Verbindungen der Formel I, in denen mindestens einer der Substituenten $Z_1$, $Z_2$, $Z_3$, $Z_4$ und $Z_5$ für Wasserstoff, $C_1$-$C_4$-Alkoxy, $C_1$-$C_4$-Alkylthio, $C_1$-$C_3$-Alkylamino oder Di-($C_1$-$C_3$-alkyl)-amino steht, eine Verbindung der Formel IX

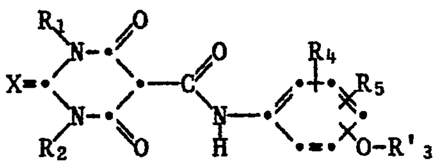

(IX)

worin $R_1$, $R_2$, $R_4$, $R_5$ und X die für Formel I angegebenen Bedeutungen haben und $R'_3$ für einen heteroaromatischen 6-gliedrigen Ring, welcher 2 bis 3 Stickstoffatome enthält oder einen benzokondensierten, heteroaromatischen Ring, welcher 1 bis 3 Stickstoffatome enthält, wobei der Ring oder der heterocyclische Teil des Ringsystems durch Halogen oder Methylsulfonyl substituiert ist, steht, mit einer Verbindung der Formel X

Z-H (X)

worin Z für $C_1$-$C_4$-Alkoxy, $C_1$-$C_4$-Alkylthio, $C_1$-$C_3$-Alkylamino oder Di-($C_1$-$C_3$-alkyl)-amino steht, umsetzt.

13. Verfahren zur Herstellung von Verbindungen der Formel VII

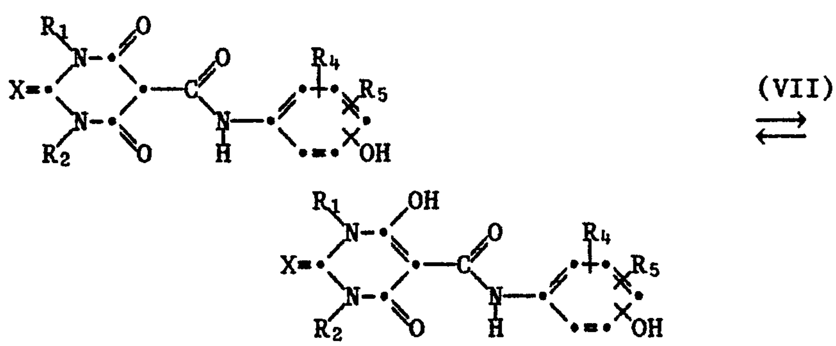

(VII)

einschliesslich ihrer tautomeren Formen und Salze, worin $R_1$ $C_1$-$C_4$-Alkyl, $C_1$-$C_3$-Alkoxy, Allyl oder $C_3$-$C_6$-Cycloalkyl,

$R_2$ $C_1$-$C_4$-Alkyl oder Allyl,

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und

X ein Sauerstoff- oder Schwefelatom bedeuten,

dadurch gekennzeichnet, dass man eine Verbindung der Formel II

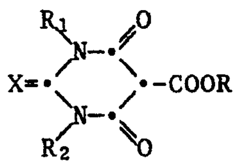

(II)

worin $R_1$, $R_2$ und X die für Formel VII angegebenen Bedeutungen haben und R $C_1$-$C_5$-Alkyl oder unsubstituiertes oder durch Nitro substituiertes Phenyl bedeutet, mit einer Verbindung der Formel XI

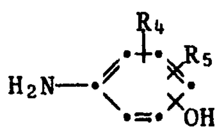

(XI)

worin $R_4$ und $R_5$ die für Formel VII angegebenen Bedeutungen haben, umsetzt.

14. Verbindungen der Formel VII

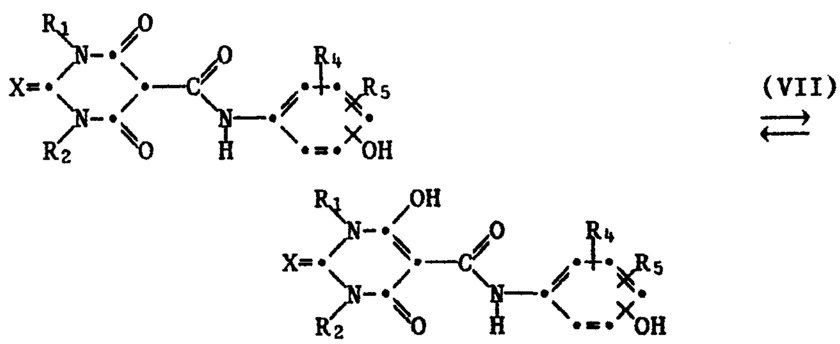

(VII)

einschliesslich ihrer tautomeren Formen und Salze, worin $R_1$, $R_2$, $R_4$, $R_5$ und X die für Formel VII im Anspruch 13 angegebenen Bedeutungen haben.

15. Verfahren zur Herstellung von Verbindungen der Formel III

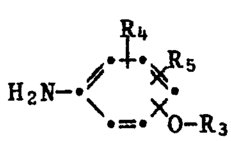

(III)

worin

$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, dadurch gekennzeichnet, dass man eine Verbindung der Formel XI

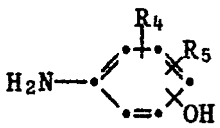

(XI)

worin $R_4$ und $R_5$ die für Formel III angegebenen Bedeutungen haben, mit einer Verbindung der Formel VIII

$Q$-$R_3$ (VIII)

worin Q für eine übliche Abgangsgruppe steht und $R_3$ die für Formel I angegebene Bedeutung hat, umsetzt.

16. Verbindungen der Formel III

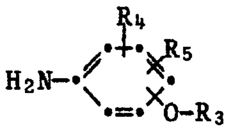

(III)

worin $R_3$, $R_4$ und $R_5$ die für Formel III im Anspruch 15 angegebenen Bedeutungen haben.

17. Anthelmintisches Mittel, dadurch gekennzeichnet, dass es als aktive Komponente mindestens eine Verbindung der Formel I, ein Tautomeres oder ein Salz davon gemäss Anspruch I neben Träger- und weiteren Hilfsstoffen enthält.

18. Mittel nach Anspruch 17, dadurch gekennzeichnet, dass es einen Wirkstoffgehalt von 0,1 bis 99,0 Gew.% und einen Gehalt an Träger- und weiteren Hilfsstoffen von 99,9 bis 1 Gew.% besitzt.

19. Verfahren zur Bekämpfung parasitärer Helminthen, dadurch gekennzeichnet, dass man einem Tier eine anthelmintisch wirksame Menge einer Verbindung der Formel I gemäss Anspruch 1 verabreicht.

20. Verwendung einer Verbindung der Formel I gemäss Anspruch 1 zur Bekämpfung von parasitären Helminthen.

FO 7.5 SES/bg*/cs*

Patentansprüche für den Vertragsstaat: AT

1. Anthelmintisches Mittel, dadurch gekennzeichnet, dass es als aktive Komponente mindestens ein 5-Phenylcarbamoylbarbitursäurederivat der allgemeinen Formel I

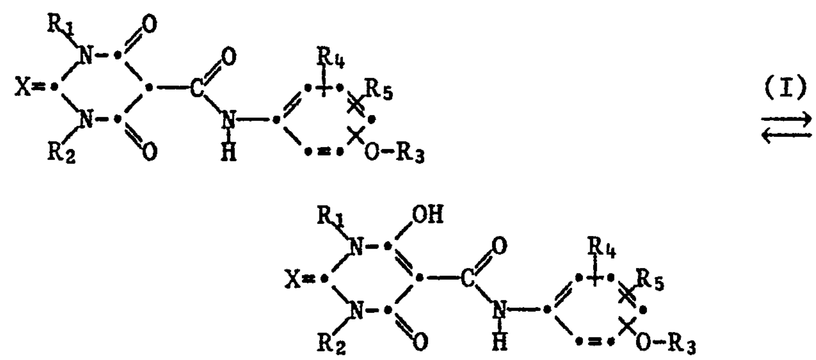

(I)

einschliesslich seiner tautomeren Formen, Salze und N-oxyde, worin
$R_1$ $C_1$-$C_4$-Alkyl, $C_1$-$C_3$-Alkoxy, Allyl oder $C_3$-$C_6$-Cycloalkyl,
$R_2$ $C_1$-$C_4$-Alkyl oder Allyl,
$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und
X ein Sauerstoff- oder Schwefelatom bedeuten,
neben Träger- und weiteren Hilfsstoffen enthält.

2. Mittel gemäss Anspruch 1, dadurch gekennzeichnet, dass es als aktive Komponente mindestens ein 5-Phenylcarbamoylbarbitursäurederivat der Formel I

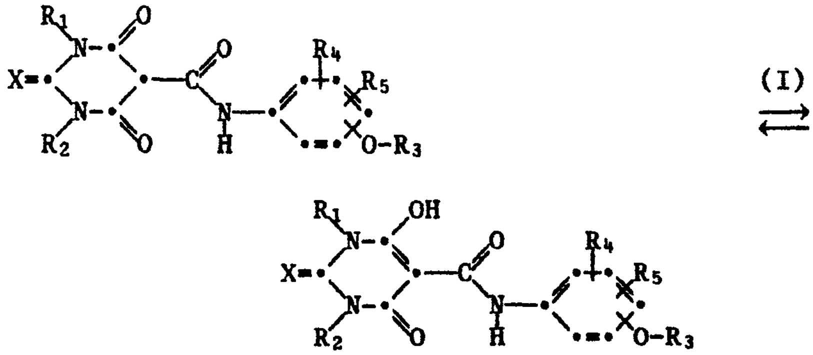

(I)

einschliesslich seiner tautomeren Formen und Salze, worin
$R_1$ $C_1$-$C_4$-Alkyl, $C_1$-$C_3$-Alkoxy, Allyl oder $C_3$-$C_6$-Cycloalkyl,
$R_2$ $C_1$-$C_4$-Alkyl oder Allyl,
$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und
X ein Sauerstoff- oder Schwefelatom bedeuten, enthält.

3. Mittel gemäss Anspruch 2, dadurch gekennzeichnet, dass es eine Verbindung der Formel I enthält, in der $R_1$, $R_2$, $R_4$, $R_5$ und X die im Anspruch 2 angegebenen Bedeutungen haben und $R_3$ für einen cyclischen Rest, ausgewählt aus der Gruppe bestehend aus

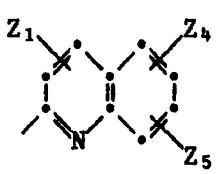
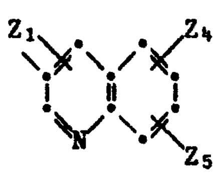
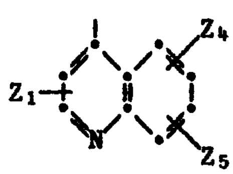
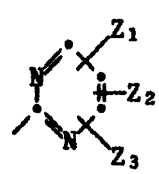

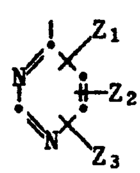
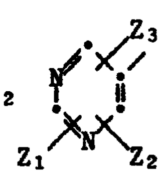
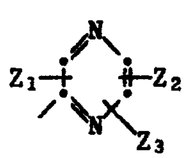
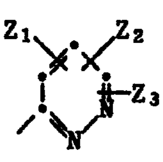
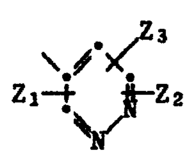

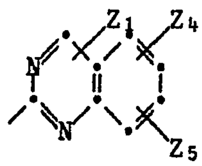
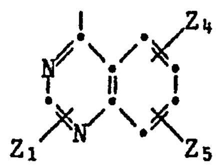
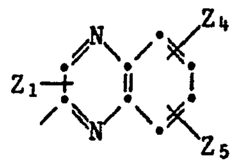
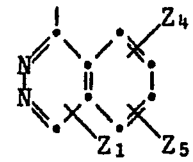

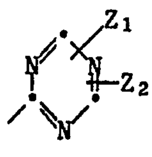
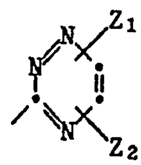
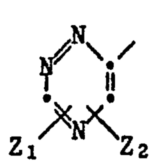
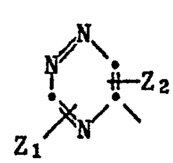
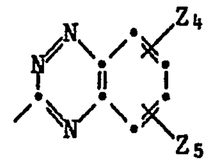

worin $Z_1$, $Z_2$, $Z_3$, $Z_4$ und $Z_5$ unabhängig voneinander Wasserstoff, $C_1$-$C_4$-Alkyl, Halogen-$C_1$-$C_4$-Alkyl, Alkoxyalkyl mit insgesamt 2 bis 4 Kohlenstoffatomen, $C_1$-$C_4$-Alkoxy, Halogen-$C_1$-$C_4$-Alkoxy, $C_1$-$C_4$-Alkylthio, Halogen-$C_1$-$C_4$-Alkylthio, $C_1$-$C_3$-Alkylamino, Di-($C_1$-$C_3$-alkyl)-amino, Allyl, Propargyl, Halogen, Nitro, Cyan, $C_3$-$C_6$-Cycloalkyl oder Phenyl bedeuten und $Z_1$, $Z_2$ und $Z_3$ am heteroaromatischen, $Z_4$ und $Z_5$ am ankondensierten homoaromatischen Ring stehen, steht.

4. Mittel gemäss Anspruch 1, dadurch gekennzeichnet, dass es eine Verbindung der Formel I enthält, in der $R_1$ $C_1$-$C_4$-Alkyl, $C_1$-$C_3$-Alkoxy, Allyl oder $C_3$-$C_6$-Cycloalkyl, $R_2$ $C_1$-$C_4$-Alkyl, $R_3$ einen unsubstituierten oder ein- bis dreifach durch Substituenten der Gruppe $C_1$-$C_4$-Alkyl, Halogen-$C_1$-$C_4$-Alkyl, $C_1$-$C_4$-Alkoxy, Halogen-$C_1$-$C_4$-Alkoxy, $C_1$-$C_4$-Alkylthio, $C_1$-$C_3$-Alkylamino, Di-($C_1$-$C_3$-alkyl)-amino, Allyl, Halogen, $C_3$-$C_6$-Cycloalkyl oder Phenyl substituierten, über ein Kohlenstoffatom gebundenen Pyrazinyl-, Pyrimidinyl-, Pyridazinyl-, Triazinyl-, Chinoxalinyl- oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten.

5. Mittel gemäss Anspruch 4, dadurch gekennzeichnet, dass es eine Verbindung der Formel I enthält, in der $R_1$ $C_1$-$C_4$-Alkyl, Allyl, Cyclopropyl oder $C_1$-$C_3$-Alkoxy, $R_2$ $C_1$-$C_4$-Alkyl, $R_3$ einen unsubstituierten oder ein-bis dreifach durch Substituenten der Gruppe $C_1$-$C_4$-Alkyl, Halogen-$C_1$-$C_4$-Alkyl, $C_1$-$C_4$-Alkoxy, $C_1$-$C_4$-Alkylthio, Di-($C_1$-$C_3$-alkyl)-amino, Halogen oder Phenyl substituierten, über ein Kohlenstoffatom gebundenen Pyrazinyl-, Pyrimidinyl-, Pyridazinyl-,

Triazinyl-, Chinoxalinyl- oder Chinolinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff-oder Schwefelatom bedeuten.

6. Mittel gemäss Anspruch 1, dadurch gekennzeichnet, dass es eine Verbindung der Formel I enthält, in der $R_1$ $C_1$-$C_4$-Alkyl, Methoxy, Allyl oder Cyclopropyl, $R_2$ Methyl, $R_3$ einen unsubstituierten oder ein- bis dreifach durch Substituenten der Gruppe $C_1$-$C_4$-Alkyl, Methoxy, Methylamino, Dimethylamino, Methylthio, Trifluormethyl, Halogen oder Phenyl substituierten Pyrazinyl-, Pyridazinyl-, Triazinyl- Pyrimidinyl-, Chinoxalinyl-, Chinolyl- oder Chinazolinylrest, $R_4$ Wasserstoff, $C_1$-$C_4$-Alkyl oder Methoxy, $R_5$ Wasserstoff oder Methyl und X ein Sauerstoff- oder Schwefelatom bedeuten.

7. Mittel gemäss Anspruch 5, dadurch gekennzeichnet, dass es eine Verbindung der Formel I enthält, in der $R_1$ $C_1$-$C_4$-Alkyl, Allyl, Cyclopropyl oder Methoxy, $R_2$ Methyl, $R_3$ einen unsubstituierten oder ein- bis dreifach durch Substituenten der Gruppe $C_1$-$C_4$-Alkyl, Trifluormethyl, Methoxy, Methylthio, Chlor oder Phenyl substituierten, über ein Kohlenstoffatom gebundenen Pyrimidinyl-, Pyrazinyl- oder Pyridazinylrest, $R_4$ Wasserstoff, $C_1$-$C_4$-Alkyl oder $C_1$-$C_4$-Alkoxy, $R_5$ Wasserstoff oder $C_1$-$C_4$-Alkyl und X ein Sauerstoff- oder Schwefelatom bedeuten und der Molekülteil $-OR_3$ in meta- oder para-Stellung zum Stickstoffatom der Carbamoylgruppe steht.

8. Mittel gemäss Anspruch 5, dadurch gekennzeichnet, dass es eine Verbindung der Formel I enthält, in der $R_1$ Methyl oder Methoxy, $R_2$ Methyl, $R_3$ einen unsubstituierten oder ein- oder zweifach durch Substituenten der Gruppe $C_1$-$C_4$-Alkyl, Halogen-$C_1$-$C_4$-Alkyl, Halogen oder Phenyl substituierten, über ein Kohlenstoffatom gebundenen Pyrimidinylring, $R_4$ Wasserstoff, $C_1$-$C_4$-Alkyl oder $C_1$-$C_4$-Alkoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht.

9. Mittel gemäss Anspruch 8, dadurch gekennzeichnet, dass es eine Verbindung der Formel I enthält, in der $R_1$ Methyl, $R_2$ Methyl, $R_3$ einen ein- oder zweifach durch Substituenten der Gruppe Methyl, Trifluormethyl, Chlor oder Phenyl substituierten, über ein Kohlenstoffatom gebundenen Pyrimidinylring, $R_4$ Wasserstoff, Methyl, Isopropyl, Methoxy oder Aethoxy, $R_5$ Wasserstoff oder Methyl und X ein Sauerstoff- oder Schwefelatom bedeuten und der Molekülteil $-OR_3$ in meta- oder para-Stellung zum Stickstoffatom der Carbamoylgruppe steht.

10. Mittel gemäss Anspruch 9, dadurch gekennzeichnet, dass es eine Verbindung der Formel I enthält, in der $R_1$ Methyl, $R_2$ Methyl, $R_3$ einen durch Trifluormethyl einfach oder durch Trifluormethyl und einen weiteren Substituenten der Gruppe Methyl, Trifluormethyl, Chlor und Phenyl insgesamt zweifach substituierten, über ein Kohlenstoffatom gebundenen Pyrimidinylring, $R_4$ Wasserstoff, Methyl, Isopropyl oder Methoxy, $R_5$ Wasserstoff oder Methyl und X ein Sauerstoff- oder Schwefelatom bedeuten und der Molekülteil $-OR_3$ in para-Stellung zum Stickstoffatom der Carbamoylgruppe steht.

11. Mittel gemäss Anspruch 1, dadurch gekennzeichnet, dass es eine Verbindung der Formel I aus der Gruppe:
1,3-Dimethyl-5-[4-(6-chlor-1-oxy-pyridazin-3-yl-oxy)-phenylcarbamoyl]-barbitursäure
1,3-Dimethyl-5-[4-(3-methylpyrazin-2-yl-oxy)-phenylcarbamoyl]-barbitursäure,
1,3-Dimethyl-5-[4-(3-chlorpyrazin-2-yl-oxy)-phenylcarbamoyl]-barbitursäure,
1,3-Dimethyl-5-[4-(3-methylpyrazin-2-yl-oxy)-phenylcarbamoyl]-2-thiobarbitursäure,
1,3-Dimethyl-5-[4-(6-trifluormethylpyrimidin-4-yl-oxy)-phenylcarbamoyl]-barbitursäure,
1,3-Dimethyl-5-[4-(4-trifluormethylpyrimidin-2-yl-oxy)-phenylcarbamoyl]-barbitursäure,
1,3-Dimethyl-5-[4-(2-trifluormethylpyrimidin-4-yl-oxy)-phenylcarbamoyl]-2-thiobarbitursäure,

1,3-Dimethyl-5-[4-methoxy-3-(6-trifluormethylpyrimidin-4-yl-oxy)-phenylcarbamoyl]-2-thiobarbitursäure,
1,3-Dimethyl-5-[3-methoxy-4-(4-trifluormethylpyrimidin-2-yl-oxy)-phenylcarbamoyl]-2-thiobarbitursäure,
1,3-Dimethyl-5-[2-isopropyl-4-(6-trifluormethylpyrimidin-4-yl-oxy)-phenylcarbamoyl]-barbitursäure,
1,3-Dimethyl-5-[2,6-dimethyl-4-(2-trifluormethyl-pyrimidin-4-yl-oxy)-phenylcarbamoyl]-barbitursäure,
1,3-Dimethyl-5-[3-methoxy-4-(2-trifluormethylpyrimidin-4-yl-oxy)-phenylcarbamoyl]-barbitursäure,
1,3-Dimethyl-5-[4-(2-methyl-6-trifluormethylpyrimidin-4-yl-oxy)-phenylcarbamoyl]-barbitursäure,
1,3-Dimethyl-5-[2-isopropyl-4-(6-trifluormethylpyrimidin-4-yl-oxy)-phenylcarbamoyl]-2-thiobarbitursäure,
1,3-Dimethyl-5-[2,6-dimethyl-4-(2-trifluormethylpyrimidin-4-yl-oxy)-phenylcarbamoyl]-2-thiobarbitursäure,
1,3-Dimethyl-5-[4-(5-chlorpyrimidin-2-yl-oxy)-phenylcarbamoyl]-barbitursäure,
1,3-Dimethyl-5-[3-methoxy-4-(5-methyl-4-trifluormethylpyrimidin-2-yl-oxy)-phenylcarbamoyl]-barbitursäure,
1,3-Dimethyl-5-[4-(5-phenyl-4-trifluormethylpyrimidin-2-yl-oxy)-phenylcarbamoyl]-barbitursäure,
1,3-Dimethyl-5-[4-(4-methyl-6-trifluormethylpyrimidin-2-yl-oxy)-phenylcarbamoyl]-barbitursäure,
1,3-Dimethyl-5-[4-(4-methyl-6-trifluormethylpyrimidin-2-yl-oxy)-phenylcarbamoyl]-2-thiobarbitursäure
1,3-Dimethyl-5-[4-(6-methyl-2-trifluormethylpyrimidin-4-yl-oxy)-phenylcarbamoyl]-barbitursäure und
1,3-Dimethyl-5-[4-(2-trifluormethylpyrimidin-4-yl-oxy)-phenylcarbamoyl]-barbitursäure enthält.

12. Mittel nach Anspruch 1, dadurch gekennzeichnet, dass es einen Wirkstoffgehalt von 0,1 bis 99,0 Gew.% und einen Gehalt an Träger- und weiteren Hilfsstoffen von 99,9 bis 1 Gew.% besitzt.

13. Verfahren zur Herstellung von Verbindungen der Formel I gemäss Anspruch 1, dadurch gekennzeichnet, dass man

a) eine Verbindung der Formel II

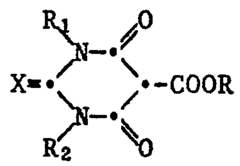

(II)

worin $R_1$, $R_2$ und X die für Formel I angegebenen Bedeutungen haben und R $C_1$-$C_5$-Alkyl oder unsubstituiertes oder durch Nitro substituiertes Phenyl bedeutet, mit einer Verbindung der Formel III

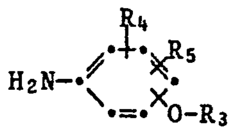

(III)

worin $R_3$, $R_4$ und $R_5$ die für Formel I angegebenen Bedeutungen haben, umsetzt, oder

b) eine Verbindung der Formel IV

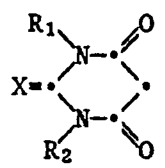

(IV)

worin $R_1$, $R_2$ und X die für Formel I angegebenen Bedeutungen haben, mit einer Verbindung der Formel V

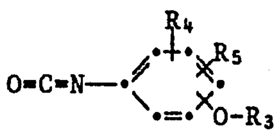

(V)

worin $R_3$, $R_4$ und $R_5$ die für Formel I angegebenen Bedeutungen haben, umsetzt, oder

c) eine Verbindung der Formel IV, worin $R_1$, $R_2$ und X die für Formel I angegebenen Bedeutungen haben, mit einer Verbindung der Formel VI

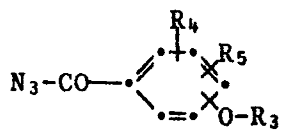

(VI)

worin $R_3$, $R_4$ und $R_5$ die für Formel I angegebenen Bedeutungen haben, umsetzt, oder

d) eine Verbindung der Formel VII

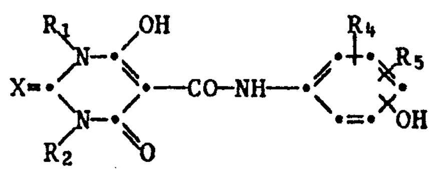

(VII)

worin $R_1$, $R_2$, $R_4$, $R_5$ und X die für Formel I angegebenen Bedeutungen haben, mit einer Verbindung der Formel VIII

$$Q-R_3 \qquad \text{(VIII)}$$

worin Q für eine übliche Abgangsgruppe steht und $R_3$ die für Formel I angegebene Bedeutung hat, in Gegenwart einer Base umsetzt, oder

e) zur Herstellung von Verbindungen der Formel I, in denen mindestens einer der Substituenten $Z_1$, $Z_2$, $Z_3$, $Z_4$ und $Z_5$ für Wasserstoff, $C_1$-$C_4$-Alkoxy, $C_1$-$C_4$-Alkylthio, $C_1$-$C_3$-Alkylamino oder Di-($C_1$-$C_3$-alkyl)-amino steht, eine Verbindung der Formel IX

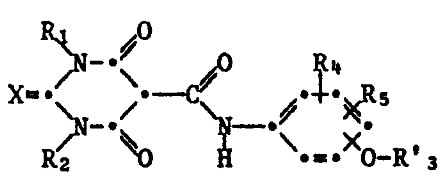

(IX)

worin $R_1$, $R_2$, $R_4$, $R_5$ und X die für Formel I angegebenen Bedeutungen haben und $R'_3$ für einen heteroaromatischen 6-gliedrigen Ring, welcher 2 bis 3 Stickstoffatome enthält oder einen benzokondensierten, heteroaromatischen Ring, welcher 1 bis 3 Stickstoffatome enthält, wobei der Ring oder der heterocyclische Teil des Ringsystems durch Halogen oder Methylsulfonyl substituiert ist, steht, mit einer Verbindung der Formel X

Z-H (X)

worin Z für $C_1$-$C_4$-Alkoxy, $C_1$-$C_4$-Alkylthio, $C_1$-$C_3$-Alkylamino oder Di-($C_1$-$C_3$-alkyl)-amino steht, umsetzt.

14. Verfahren zur Herstellung von Verbindungen der Formel VII

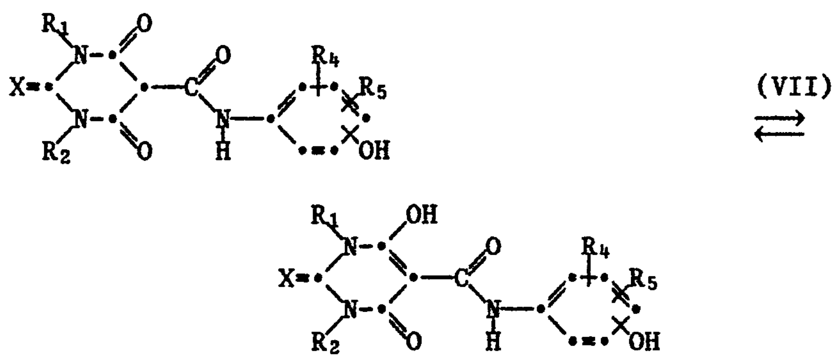

(VII)

einschliesslich ihrer tautomeren Formen und Salze, worin
$R_1$ $C_1$-$C_4$-Alkyl, $C_1$-$C_3$-Alkoxy, Allyl oder $C_3$-$C_6$-Cycloalkyl,
$R_2$ $C_1$-$C_4$-Alkyl oder Allyl,
$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl und
X ein Sauerstoff- oder Schwefelatom bedeuten,
dadurch gekennzeichnet, dass man eine Verbindung der Formel II

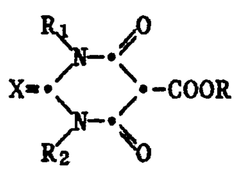

(II)

worin $R_1$, $R_2$ und X die für Formel VII angegebenen Bedeutungen haben und R $C_1$-$C_5$-Alkyl oder unsubstituiertes oder durch Nitro substituiertes Phenyl bedeutet, mit einer Verbindung der Formel XI

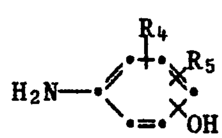

(XI)

worin $R_4$ und $R_5$ die für Formel VII angegebenen Bedeutungen haben, umsetzt.

15. Verfahren zur Herstellung von Verbindungen der Formel III

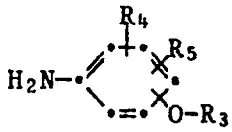

(III)

worin

$R_3$ einen unsubstituierten oder substituierten heteroaromatischen 6-gliederigen Ring, welcher 2 oder 3 Stickstoffatome enthält oder einen benzokondensierten, unsubstituierten oder substituierten heteroaromatischen 6-gliederigen Ring, welcher 1 bis 3 Stickstoffatome enthält,

$R_4$ und $R_5$ unabhängig voneinander Wasserstoff, $C_1$-$C_4$-Alkyl, $C_1$-$C_4$-Alkoxy oder Halogen-$C_1$-$C_3$-Alkyl bedeuten, dadurch gekennzeichnet, dass man eine Verbindung der Formel XI

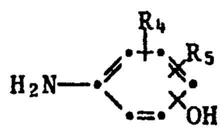

(XI)

worin $R_4$ und $R_5$ die für Formel III angegebenen Bedeutungen haben, mit einer Verbindung der Formel VIII

$Q-R_3$ (VIII)

worin Q für eine übliche Abgangsgruppe steht und $R_3$ die für Formel I angegebene Bedeutung hat, umsetzt.

16. Verfahren zur Bekämpfung parasitärer Helminthen, dadurch gekennzeichnet, dass man einem Tier eine anthelmintisch wirksame Menge einer Verbindung der Formel I gemäss Anspruch 1 verabreicht.

17. Verwendung einer Verbindung der Formel I gemäss Anspruch 1 zur Bekämpfung von parasitären Helminthen.

FO 7.5 SES/cw*/cs*

Europäisches Patentamt

**EUROPÄISCHER TEILRECHERCHENBERICHT,** der nach Regel 45 des Europäischen Patentübereinkommens für das weitere Verfahren als europäischer Recherchenbericht gilt

0 191 474

EP 86101771.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 2 719 777 (CIBA)<br>* Formel I *<br>-- | 1 | C 07 D 403/12<br>A 61 K 31/515 |
| A | DE - A1 - 2 936 457 (CIBA)<br>* Formel 1 *<br>-- | 1 | |
| A | DE - A1 - 2 641 979 (JCJ)<br>* Anspruch 1 *<br>-- | 1 | |

RECHERCHIERTE SACHGEBIETE (Int. Cl.4)

C 07 D 403/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-14,17,18

Unvollständig recherchierte Patentansprüche: –

Nicht recherchierte Patentansprüche: 19,20

Grund für die Beschränkung der Recherche:

Art. 52(4), Verfahren zur therapeutischen Behandlung des menschlichen oder tierischen Körpers

| Recherchenort | Abschlußdatum der Recherche | Prüfer |
|---|---|---|
| WIEN | 23-04-1986 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

Europäisches Patentamt

# EUROPÄISCHER TEILRECHERCHENBERICHT

0 191 474

EP 86101771.3

| EINSCHLÄGIGE DOKUMENTE | | | KLASSIFIKATION DER ANMELDUNG (Int. Cl. 4) |
|---|---|---|---|
| Kategorie | Kennzeichnung des Dokuments mit Angabe, soweit erforderlich, der maßgeblichen Teile | betrifft Anspruch | |
| A | CHEMICAL ABSTRACTS, Band 99, Nr. 10, 5. September 1983, Columbus, Ohio, USA<br>DE SOUSA, BERNARDO; SCHMID, WERNER; HEFTI, HEINZ; BELLUS, DANIEL "A biological evaluation of pyrethroids as potential mothproofing agents. Part 3. A complete and highly effective mothproofing agent comprising a pyrethroid and a hexahydropyrimidine"<br>Seite 63, Spalte 2, Zusammenfassung-Nr. 72 077x<br>& J. Soc. Dyers Colour. 1983, 99(4), 118-21<br>---- | 1 | |
| | | | RECHERCHIERTE SACHGEBIETE (Int. Cl.4) |

EPA Form 1505.3 06.78